# EUROPEAN PATENT SPECIFICATION

(11) **EP 2 519 625 B1**
(45) Date of publication and mention of the grant of the patent: **26.09.2018**
(21) Application number: 10841651.2
(22) Date of filing: 28.12.2010
(51) Int. Cl.: C12N 1/10

(54) **RECOMBINANT THRAUSTOCHYTRIDS THAT GROW ON SUCROSE, AND COMPOSITIONS, METHODS OF MAKING, AND USES THEREOF**
AUF SACCHAROSE WACHSENDE REKOMBINANTE THRAUSTOCHYTRIDE SOWIE ZUSAMMENSETZUNGEN, HERSTELLUNGSVERFAHREN UND VERWENDUNGEN DAVON
THRAUSTOCHYTRIDES RECOMBINANTS QUI SE DÉVELOPPENT SUR LE SACCHAROSE, COMPOSITIONS LES CONTENANT, LEURS PROCÉDÉS DE PRÉPARATION, ET LEURS UTILISATIONS

(30) Priority: 28.12.2009 US 290443 P
(43) Date of publication of application: 07.11.2012
(73) Proprietor: DSM IP Assets B.V., 6411 TE Heerlen (NL)
(72) Inventor: LIPPMEIER, James Casey, Columbia, Maryland 21045 (US); APT, Kirk E., Ellicott City, Maryland 21042 (US); HANSEN, Jon Milton, Lexington, Kentucky 40509 (US)
(74) Representative: D Young & Co LLP
(86) International application number: PCT/US2010/062277
(87) International publication number: WO 2011/082190

(56) References cited:
- WO-A1-2011/026008
- WO-A2-2008/151149
- WO-A2-2009/126843
- US-A- 5 576 428
- US-A1- 2003 166 207
- TAOKA YOUSUKE ET AL: "Extracellular Enzymes Produced by Marine Eukaryotes, Thraustochytrids", BIOSCIENCE BIOTECHNOLOGY AND BIOCHEMISTRY, vol. 73, no. 1, January 2009 (2009-01), pages 180-182, XP002698981, ISSN: 0916-8451
- CHATDUMRONG ET AL.: 'Optimization of Docosahexaenoic Acid (DHA) Production and Improvement of Astaxanthin Content in a Mutant Schizochytrium limacinum Isolated from Mangrove Forest' NAT. SCI. vol. 41, 2007, THAILAND., pages 324 - 334
- LI ET AL.: 'Screening and Characterization of Squalene-Producing Thraustochytrids' J. AGRIC. FOOD CHEM. vol. 57, no. 10, 17 April 2009, HONG KONG MANGROVES, pages 4267 - 4272

## Description

### BACKGROUND OF THE INVENTION

### Field of the Invention

The present invention is directed to recombinant thraustochytrids that grow on sucrose and cell cultures comprising the recombinant thraustochytrids, as well as methods of producing cell cultures, biomasses, microbial oils, and compositions using the recombinant thraustochytrids.

### Background

Fatty acids are classified based on the length and saturation characteristics of the carbon chain. Fatty acids are termed short chain, medium chain, or long chain fatty acids based on the number of carbons present in the chain, are termed saturated fatty acids when no double bonds are present between the carbon atoms, and are termed unsaturated fatty acids when double bonds are present. Unsaturated long chain fatty acids are monounsaturated when only one double bond is present and are polyunsaturated when more than one double bond is present.

Polyunsaturated fatty acids (PUFAs) are classified based on the position of the first double bond from the methyl end of the fatty acid: omega-3 (n-3) fatty acids contain a first double bond at the third carbon, while omega-6 (n-6) fatty acids contain a first double bond at the sixth carbon. For example, docosahexaenoic acid ("DHA") is an omega-3 long chain polyunsaturated fatty acid (LC-PUFA) with a chain length of 22 carbons and 6 double bonds, often designated as "22:6 n-3." Other omega-3 LC-PUFAs include eicosapentaenoic acid ("EPA"), designated as "20:5 n-3," and omega-3 docosapentaenoic acid ("DPA n-3"), designated as "22:5 n-3." DHA and EPA have been termed "essential" fatty acids. Omega-6 LC-PUFAs include arachidonic acid ("ARA"), designated as "20:4 n-6," and omega-6 docosapentaenoic acid ("DPA n-6"), designated as "22:5 n-6."

Omega-3 fatty acids are biologically important molecules that affect cellular physiology due to their presence in cell membranes, regulate production and gene expression of biologically active compounds, and serve as biosynthetic substrates. Roche, H. M., Proc. Nutr. Soc. 58: 397-401 (1999). DHA, for example, accounts for approximately 15%-20% of lipids in the human cerebral cortex and 30%-60% of lipids in the retina, is concentrated in the testes and sperm, and is an important component of breast milk. Jean-Pascal Bergé & Gilles Barnathan, Fatty Acids from Lipids of Marine Organisms: Molecular Biodiversity, Roles as Biomarkers, Biologically Active Compounds, and Economical Aspects, in Marine Biotechnology I 49 (T. Scheper, ed., 2005). DHA accounts for up to 97% of the omega-3 fatty acids in the brain and up to 93% of the omega-3 fatty acids in the retina. Moreover, DHA is essential for both fetal and infant development as well as maintenance of cognitive functions in adults. *Id.* Omega-3 fatty acids, including DHA and EPA, also possess anti-inflammatory properties. *See,* e.g., *id.* and Simopoulos, A.P., J. Am. Coll. Nutr. 21: 495-595 (2002). In particular, EPA competes with arachidonic acid for synthesis of eicosanoids such as prostaglandins and leukotrienes through cyclooxygenases and lipoxygenases. *Id.* Because omega-3 fatty acids are not synthesized de novo in the human body, these fatty acids must be derived from nutritional sources.

Thraustochytrids, which are microalgal organisms of the order Thraustochytriales, are recognized as alternative sources for the production of lipids. For example, strains of thraustrochytrid species have been reported to produce omega-3 fatty acids as a high percentage of the total fatty acids produced by the organisms. *See,* e.g., U.S. Patent No. 5,130,242; Huang, J. et al., J. Am. Oil. Chem. Soc. 78: 605-610 (2001); and Huang, J. et al., Mar. Biotechnol. 5: 450-457 (2003). Thraustochytrids have also been recognized as sources of lipids for the production of biofuels. *See,* e.g., U.S. Publ. No. 2009/0064567 and WO 2008/067605.

While thraustochytrids metabolize glucose and/or fructose, which are the two sugar components of sucrose, they do not appear to naturally metabolize sucrose. *See,* e.g., Aki et al., JAOCS 80:789-794 (2003); Yokochi et al., Appl. Microbiol. Biotechnol. 49:72-76 (1998); Honda et al., Mycol. Res. 4:439-448 (1998); Singh, World J. of Microbiology 12:76-81 (1996); Goldstein, American J. of Botany 50:271-279 (1963); and U.S. Pat. No. 5,340,742. As such, thraustochytrid cultures, including commercial and industrial cultures, currently require glucose syrups as carbon and energy sources, rather than more inexpensive carbohydrate sources containing sucrose.

A continuing need exists for producing thraustochytrids with the ability to grow on alternative carbon sources such as sucrose for use in commercial and industrial applications.

### BRIEF SUMMARY OF THE INVENTION

The present invention is directed to a method of producing a thraustochytrid cell culture, comprising: (a) transforming a thraustochytrid cell with a nucleic acid molecule comprising a polynucleotide sequence encoding a heterologous invertase, wherein the invertase is bound to the plasma membrane of the thraustochytrid cell, and (b) growing the transformed thraustochytrid cell in a culture medium comprising sucrose as a carbon source. In some embodiments, the invertase is operably linked to a signal peptide. In some embodiments, the polynucleotide sequence encoding the heterologous invertase is at least 90% identical to the polynucleotide sequence of SEQ ID NO: 1. In some embodiments, the method further comprises transforming the thraustochytrid cell with a nucleic acid molecule comprising a polynucleotide sequence encoding a heterologous sucrose transporter. In some embodiments, the polynucleotide sequence encoding the heterologous sucrose transporter is at least 90% identical to the polynucleotide sequence of Accession No. L47346. In some embodiments, the thraustochytrid is a *Schizochytrium* or a
*Thraustochytrium.*

The present invention is also directed to a thraustochytrid cell comprising a nucleic acid molecule comprising a polynucleotide sequence encoding a heterologous invertase, wherein the invertase is bound to the plasma membrane of the thraustochytrid cell. In some embodiments, the invertase is operably linked to a signal peptide. In some embodiments, the polynucleotide sequence encoding the heterologous invertase is at least 90% identical to the polynucleotide sequence of SEQ ID NO: 1. In some embodiments, the cell further comprises a polynucleotide sequence encoding a heterologous sucrose transporter. In some embodiments, the polynucleotide sequence encoding the heterologous sucrose transporter is at least 90% identical to the polynucleotide sequence of Accession No. L47346. In some embodiments, the thraustochytrid is a *Schizochytrium* or a *Thraustochytrium.*

The present invention is also directed to a thraustochytrid culture comprising: (a) any of the thraustochytrid cells of the invention, and (b) a cell culture medium comprising sucrose as a carbon source.

The present invention is also directed to a method of producing a thraustochytrid biomass, comprising: (a) growing any of the thraustochytrid cells of the invention in a culture medium comprising sucrose as a carbon source, and (b) harvesting the biomass from the culture medium.

The present invention is also directed to a method of producing a microbial oil, comprising: (a) growing any of the thraustochytrid cells of the invention in a culture medium comprising sucrose as a carbon source to produce a biomass, and (b) extracting an oil from the biomass.

The present invention is also directed to a method of producing a food product, cosmetic, industrial composition, or pharmaceutical composition for an animal or human, comprising: (a) growing any of the thraustochytrid cells of the invention in a culture medium comprising sucrose as a carbon source, (b) harvesting a biomass from the culture medium, and (c) preparing the food product, cosmetic, industrial composition, or pharmaceutical composition from the biomass. In some embodiments, the method further comprises extracting an oil from the biomass and preparing the food product, cosmetic, industrial composition, or pharmaceutical composition from the oil. In some embodiments, the food product is an infant formula. In some embodiments, the food product is milk, a beverage, a therapeutic drink, a nutritional drink, or a combination thereof. In some embodiments, the food product is an additive for animal or human food. In some embodiments, the food product is a nutritional supplement. In some embodiments, the food product is an animal feed.

The invention is defined by the claims. Subject-matter outside the scope of the claims is provided for information only.

### BRIEF DESCRIPTION OF THE DRAWINGS/FIGURES

**FIG. 1** shows a codon usage table for *Schizochytrium.*
**FIG. 2** shows a plasmid map of pSchiz-EPCT(+)-s1Suc2_CL0076, also termed pCL0076 (SEQ ID NO:2).
**FIG. 3** shows dry weight (g/L) of cell pellets from cultures of *Schizochytrium* sp. ATCC 20888 transformed with pCL0076 grown on sucrose-SSFM. The transformants are referred to as 1-1, 1-3, 1-24, 3-1, 3-2, 3-5, 3-21, 4-1, 4-24, and 4-31. "Control" refers to wild-type *Schizochytrium* sp. ATCC 20888 cells grown on glucose-SSFM.
**FIG. 4** shows fat content (expressed as % weight of the dry biomass) in cell pellets from cultures of *Schizochytrium* sp. ATCC 20888 transformed with pCL0076 grown on sucrose-SSFM. The transformants are referred to as 1-1, 1-3, 1-24, 3-1, 3-2, 3-5, 3-21, 4-1, 4-24, and 4-31. "Control" refers to wild-type *Schizochytrium* sp. ATCC 20888 cells grown on glucose-SSFM.
**FIG. 5** shows dry weight (g/L) of cell pellets measured over time (days) for cultures of *Schizochytrium* sp. ATCC 20888 transformed with pCL0076 grown on sucrose-SSFM. The transformants are referred to as 1-3 and 3-5. "Control" refers to wild-type *Schizochytrium* sp. ATCC 20888 cells grown on glucose-SSFM.
**FIG. 6** shows fat content (expressed as % weight of the dry biomass) in cell pellets from cultures of two transformants grown on sucrose-SSFM. The transformants are referred to as 1-3 and 3-5. "Control" refers to wild-type *Schizochytrium* sp. ATCC 20888 cells grown on glucose-SSFM.
**FIG. 7** shows dry weight (g/L) of cell pellets from cultures of *Schizochytrium* strain B76-32 transformed with pCL0076 and harvested after either 2 days or 7 days of growth in sucrose-SSFM. 2118* refers to a sub-isolate of wild-type *Schizochytrium* sp. ATCC 20888 cells grown on glucose-SSFM. B76-32** refers to the B76-32 parent strain grown on glucose-SSFM.

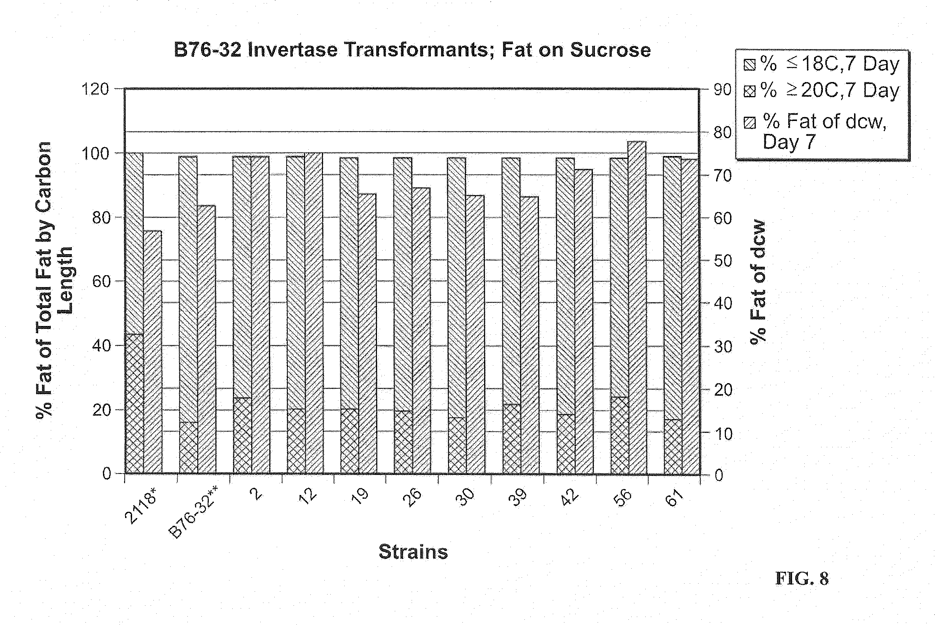

**FIG. 8** shows fat content of cell pellets from cultures of *Schizochytrium* strain B76-32 transformed with pCL0076 and harvested after either 2 days or 7 days of growth in sucrose-SSFM. The rightmost column for each sample shows fat content as % weight of the dry biomass. The leftmost column for each sample shows % of total fat composed of acyl groups with 18 or fewer carbons (light grey) or 20 or more carbons (medium grey). 2118* refers to a sub-isolate of wild-type *Schizochytrium* sp. ATCC 20888 cells grown on glucose-SSFM. B76-32** refers to the B76-32 parent strain grown on glucose-SSFM.
**FIG. 9A** shows a Western blot for invertase protein and **FIG. 9B** shows a corresponding Coomassie-stained SDS-PAGE gel. *S. cerevisiae* invertase control and supernatants of a 3-day culture of pCL0076 transformant 1-3 were loaded in amounts of 5 µg, 2.5 µg, 1.25 µg, and 0.625 µg, respectively, as indicated at the top of the Western blot.
**FIG. 10A** shows an invertase activity assay illustrated by the reaction rate as a function of sucrose concentration. **FIG. 10B** shows a standard Lineweaver-Burk plot used to calculate the Kₘ and Vₘₐₓ.
**FIG. 11** shows N-glycan structures detected on *Schizochytrium* secreted proteins as determined by NSI-Total Ion Mapping of permethylated N-glycans.
**FIG. 12** shows a table of glycan species obtained by NSI-Total Ion Mapping of permethylated N-glycans.
**FIG. 13** shows a plasmid map of pCL0120 (SEQ ID NO:5).
**FIG. 14** shows a codon-optimized nucleic acid sequence (SEQ ID NO:4) encoding the Sec1 signal peptide from *Schizochytrium* fused to the mature Suc1 invertase from *Aspergillus niger* (GenBank Accession No. S33920).
**FIG. 15** shows a plasmid map of pCL0137_EPCT(+)-s1Suc1, also termed pCL0137.

### DETAILED DESCRIPTION OF THE INVENTION

The present invention is directed to recombinant thraustochytrids that grow on sucrose and cell cultures comprising the recombinant thraustochytrids, as well as methods of producing cell cultures, biomasses, microbial oils, and compositions using the recombinant thraustochytrids.

### Thraustochytrids

According to the present invention, the term "thraustochytrid" refers to any member of the order Thraustochytriales, which includes the family Thraustochytriaceae. The current taxonomic placement of the thraustochytrids can be summarized as follows:
Realm: Stramenopila (Chromista)
Phylum: Labyrinthulomycota (Heterokonta)
Class: Labyrinthulomycetes (Labyrinthulae)
Order: Labyrinthulales
   Family: Labyrinthulaceae
Order: Thraustochytriales
   Family: Thraustochytriaceae

For purposes of the present invention, strains described as thraustochytrids include the following organisms: Order: Thraustochytriales; Family: Thraustochytriaceae; Genera: *Thraustochytrium* (Species: sp., *arudimentale, aureum, benthicola, globosum, kinnei, motivum, multirudimentale, pachydermum, proliferum, roseum, striatum), Ulkenia* (Species: sp., *amoeboidea, kerguelensis, minuta, profunda, radiata, sailens, sarkariana, schizochytrops, visurgensis, yorkensis), Schizochytrium* (Species: sp., *aggregatum, limnaceum, mangrovei, minutum, octosporum), Japonochytrium* (Species: sp., *marinum*), *Aplanochytrium* (Species: sp., *haliotidis, kerguelensis, profunda, stocchinoi*)*, Althornia* (Species: sp., *crouchii*), or *Elina* (Species: sp., *marisalba, sinorifica*)*.* For the purposes of this invention, species described within *Ulkenia* will be considered to be members of the genus *Thraustochytrium. Aurantiochytrium, Oblongichytrium, Botryochytrium, Parietichytrium,* and *Sicyoidochytrium* are additional genuses encompassed by the present invention.

Strains of *Thraustochytriales* include, but are not limited to, deposited strains PTA-10212, PTA-10213, PTA-10214, PTA-10215, PTA-9695, PTA-9696, PTA-9697, PTA-9698, PTA-10208, PTA-10209, PTA-10210, PTA-10211, *Thraustochytrium* sp. (23B) (ATCC 20891); *Thraustochytrium striatum* (Schneider) (ATCC 24473); *Thraustochytrium aureum* (Goldstein) (ATCC 34304); *Thraustochytrium roseum* (Goldstein) (ATCC 28210); and *Japonochytrium* sp. (L1) (ATCC 28207). *Schizochytrium* include, but are not limited to *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited strain ATCC 28209 and deposited *Schizochytrium limacinum* strain IFO 32693.

In some embodiments, the thraustochytrid cell of the invention is a *Schizochytrium* or a *Thraustochytrium* cell. In some embodiments, the thraustochytrid is from a species selected from *Schizochytrium* sp., *Schizochytrium aggregatum, Schizochytrium limacinum, Schizochytrium minutum, Thraustochytrium* sp., *Thraustochytrium striatum, Thraustochytrium aureum, Thraustochytrium roseum, Japonochytrium* sp., and strains derived therefrom.

According to the present invention, the term "transformation" is used to refer to any method by which a nucleic acid molecule (i.e., a recombinant nucleic acid molecule) can be inserted into a thraustochytrid cell of the invention. In microbial systems, the term "transformation" is used to describe an inherited change due to the acquisition of exogenous nucleic acids by the microorganism and is essentially synonymous with the term "transfection." Suitable transformation techniques for introducing nucleic acid molecules into thraustochytrid cells include, but are not limited to, particle bombardment, electroporation, microinjection, lipofection, adsorption, infection, and protoplast fusion.

Although the phrase "nucleic acid molecule" primarily refers to the physical nucleic acid molecule and the phrases "nucleic acid sequence" or "polynucleotide sequence" primarily refers to the sequence of nucleotides in the nucleic acid molecule, the phrases are used interchangeably, especially with respect to a nucleic acid molecule, polynucleotide sequence, or a nucleic acid sequence that is capable of encoding a protein. In some embodiments, isolated nucleic acid molecules as described herein are produced using recombinant DNA technology (e.g., polymerase chain reaction (PCR) amplification or cloning) or chemical synthesis. In accordance with the present invention, an "isolated" nucleic acid molecule is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the nucleic acid molecule has been purified, but indicates that the molecule does not include an entire genome or an entire chromosome in which the nucleic acid molecule is found in nature. An isolated nucleic acid molecule can include DNA, RNA (e.g., mRNA), or derivatives of either DNA or RNA (e.g., cDNA). Isolated nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications provide the desired effect on sequence, function, and/or the biological activity of the encoded peptide or protein.

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10, or 20 amino acids. In some embodiments, a polypeptide as described herein is a heterologous polypeptide. The term "heterologous" as used herein refers to a polypeptide (or polynucleotide) sequence, for example, that is not naturally found in the thraustochytrid cell of the invention.

*Schizochytrium* naturally uses hexose sugars and glycerol as a carbon source instead of sucrose. Examination of the genome database of *Schizochytrium* did not reveal genes required for the initial steps of sucrose catabolism, supporting the general finding that thraustochytrids metabolize glucose and/or fructose, which are the two sugar components of sucrose, but do not naturally metabolize sucrose. *See also,* e.g., Aki et al., JAOCS 80:789-794 (2003); Yokochi et al., Appl. Microbiol. Biotechnol. 49:72-76 (1998); Honda et al., Mycol. Res. 4:439-448 (1998); Singh, World J. of Microbiology 12:76-81 (1996); Goldstein, American J. of Botany 50:271-279 (1963); and U.S. Pat. No. 5,340,742.

The present invention is directed to a thraustochytrid cell transformed with a nucleic acid molecule comprising a polynucleotide sequence encoding a heterologous polypeptide associated with sucrose metabolism. The polypeptide is a heterologous polypeptide. A thraustochytrid cell of the invention comprises one or more nucleic acid molecules comprising one or more polynucleotide sequences encoding one or more heterologous sucrases, comprising an invertase. In some embodiments, the thraustochytrid cell comprises a nucleic acid molecule comprising a polynucleotide sequence encoding a secreted invertase. Described herein, the thraustochytrid cell comprises a nucleic acid molecule comprising a polynucleotide sequence encoding a cytoplasmic sucrase. Described herein, the thraustochytrid cell comprises a nucleic acid molecule comprising a polynucleotide sequence encoding a sucrose transporter. In some embodiments, the thraustochytrid cell comprises one or more nucleic acid molecules comprising one or more polynucleotides encoding one or more heterologous sucrases and a heterologous sucrose transporter. Described herein, the thraustochytrid cell comprises one or more nucleic acid molecules comprising one or more polynucleotides encoding a heterologous cytoplasmic invertase and a heterologous sucrose transporter. In some embodiments, the thraustochytrid cell comprises one or more nucleic acid molecules comprising one or more polynucleotides encoding a heterologous secreted invertase and a heterologous sucrose transporter. In some embodiments, the thraustochytrid cell comprises one or more nucleic acid molecules comprising one or more polynucleotides encoding a heterologous cytoplasmic invertase, a heterologous secreted invertase, and a heterologous sucrose transporter. In some embodiments, the polynucleotide sequence is codon-optimized to maximize translation efficiency in the thraustochytrid cell. In some embodiments, the polynucleotide sequence encoding the sucrase, sucrose transporter, or combination thereof is integrated into the genome of the thraustochytrid cell. In some embodiments, the polynucleotide sequence encoding the sucrase, sucrose transporter, or combination thereof is stably integrated into the genome of the thraustochytrid cell. Sucrases are part of a family of enzymes known as glycoside hydrolases (also termed glycosidases) that catalyze the hydrolysis of glycosidic linkages to generate two smaller sugars. As described herein, a "sucrase" is any enzyme that catalyzes the hydrolysis of sucrose (also known as saccharose or table sugar) to fructose and glucose. Described herein, the sucrase may be a sucrase-isomaltase, a levanase, sacridase, beta-fructosidase, beta-fructofuranosidases, fructohydrolase, saccharase, inulinase, sucrose alpha-glucohydrolase, alpha-D-glucosidase, or a combination thereof. In the method of the invention the sucrase is an invertase. In some embodiments the sucrase is the invertase SUC2 from *Saccharomyces cerevisiae;* the invertase SacC from *Zymomonas*; the invertase INV1 from *Pichia;* the invertase INV from *Debaryomyces*; an invertase from other fungal, protist, algal, plant, or eukaryotic sources; or any combinations thereof. In certain embodiments, the sucrase is from a prokaryotic source such as coryneform bacteria or *Escherichia coli.* In some embodiments, a sucrase expressed in a thraustochytrid cell of the invention comprises a different glycosylation pattern than when expressed in the organism from which the sucrase is derived. In some embodiments, the sucrase that is expressed in the thraustochytrid cell is a glycoprotein comprising high-mannose oligosaccharides. In some embodiments, the thraustochytrid cell also comprises a polynucleotide sequence encoding an enzyme required for saccharification of a cellulose feedstock (e.g., sugar cane). *See,* e.g., U.S. 2009/0064567. In some embodiments, the thraustochytrid cell also comprises a polynucleotide sequence encoding a fructokinase, a glucokinase, a hexokinase, or a combination thereof.

In some embodiments, the expression system used for the production of a sucrase, a sucrose transporter, or a combination thereof in a thraustochytrid cell comprises regulatory elements that are derived from thraustochytrid sequences. In some embodiments, the expression system used for the production of a sucrase, a sucrose transporter, or a combination thereof in a thraustochytrid cell comprises regulatory elements that are derived from non-thraustochytrid sequences, including sequences derived from non-thraustochytrid algal sequences. In some embodiments, the expression system comprises a polynucleotide sequence encoding a sucrase, a sucrose transporter, or the combination of the two wherein the polynucleotide sequence is operably linked to any promoter sequence, any terminator sequence, and/or any other regulatory sequence that is functional in a thraustochytrid cell. Described herein, the expression system comprises polynucleotide sequences encoding a cytoplasmically expressed sucrase and a sucrose transporter. Inducible or constitutively active regulatory sequences can be used. Regulatory sequences include but are not limited to the *Schizochytrium* regulatory sequences described in U.S. Publ. No. 2010/0233760, U.S. Patent No. 7,001,772, and U.S. Publ. Nos. 2006/0275904 and 2006/0286650, such as: an OrfC promoter, an OrfC terminator, an EF1 short promoter, EF1 long promoter, a Sec1 promoter, 60S short promoter, 60S long promoter, an acetolactate synthase promoter, an acetolactate synthase terminator, an α-tubulin promoter, a promoter from a polyketide synthase (PKS) system, a fatty acid desaturase promoter, an actin promoter, an actin terminator, an elongation factor 1 alpha (ef1α) promoter, an ef1α terminator, a glyceraldehyde 3-phosphate dehydrogenase (gapdh) promoter, a gapdh terminator, and combinations thereof.

In some embodiments, the sucrase is secreted into the fermentation medium by a recombinant thraustochytrid transformed with a sucrase gene. In some embodiments, the sucrase is a secreted protein comprising a signal peptide. In some embodiments, the signal peptide is naturally associated with the exogenous sucrase (i.e., the signal peptide is the native signal sequence of the exogenous sucrase). In some embodiments, the signal peptide is not naturally associated with the exogenous sucrase but is instead a heterologous signal peptide, such as a signal peptide from a microorganism of the phylum Labyrinthulomycota. In some embodiments, the signal peptide is from a thraustochytrid. In some embodiments, the signal peptide is from a *Schizochytrium* or a *Thraustochytrium.* In some embodiments, the signal peptide is the *Schizochytrium* Sec1 signal peptide (SEQ ID NO:3). A "signal peptide" includes full-length peptides and functional fragments thereof, fusion peptides, and homologues of a naturally occurring signal peptide. A homologue of a signal peptide differs from a naturally occurring signal peptide in that at least one or a few, but not limited to one or a few, amino acids have been deleted (e.g., a truncated version of the protein, such as a peptide or fragment), inserted, inverted, substituted, and/or derivatized (e.g., by glycosylation, phosphorylation, acetylation, acylation (e.g., myristoylation and palmitoylation), prenylation, amidation, and/or addition of glycosylphosphatidyl inositol). In some embodiments, homologues of a signal peptide retain activity as a signal at least in a thraustochytrid, although the activity can be increased, decreased, or made dependent upon certain stimuli. In some embodiments, the signal peptide sequence includes, but is not limited to, a *Schizochytrium* sequence described in U.S. Publ. No. 2010/0233760, such as: a Na/Pi transporter signal peptide, a shortened Na/Pi transporter signal peptide, an alpha-1,6-mannosyltransferase (ALG12) signal peptide, a binding immunoglobulin protein (BiP) signal peptide, an alpha-1,3-glucosidase (GLS2) signal peptide, an alpha-1,3-1,6-mannosidase-like signal peptide, an alpha-1,3-1,6-mannosidase-like #1 signal peptide, an alpha-1,2-mannosidase-like signal peptide, a beta-xylosdiase-like signal peptide, a carotene synthase signal peptide, and a Sec1 signal peptide.

Described herein, the sucrase is expressed cytoplasmically along with expression of a sucrose transporter. In some embodiments, the sucrase is membrane bound, e.g., sucrase-isomaltase, which has an N-terminal membrane spanning domain (signal anchor). In certain embodiments, the sucrase is localized to the exterior of the plasma membrane. Described herein, the sucrase is fused to a membrane spanning domain.

In some embodiments, an expression cassette is used for the expression of a sucrase, a sucrose transporter, or a combination thereof in a thraustochytrid cell. The design and construction of expression cassettes use standard molecular biology techniques known to persons skilled in the art. *See,* e.g., Sambrook et al., 2001, Molecular Cloning: A Laboratory Manual, 3rd edition. In some embodiments, the thraustochytrid cell comprises an expression cassette containing genetic elements, such as at least the following operably linked in such a way that they are functional in the thraustochytrid cell: a promoter; a coding sequence comprising a sucrase, a sucrose transporter, or a combination thereof;, and a terminator region. In some embodiments, the expression cassette further comprises a polynucleotide sequence encoding a signal peptide or a membrane domain operably linked to the sucrase or sucrose transporter. The term "membrane domain" as used herein refers to any domain within a polypeptide that targets the polypeptide to a membrane and/or allows the polypeptide to maintain association with a membrane and includes, but is not limited to, a transmembrane domain (e.g., a single or multiple membrane spanning region), an integral monotopic domain, a signal anchor sequence, an ER signal sequence, an N-terminal or internal or C-terminal stop transfer signal, a glycosylphosophatidylinositol anchor, and combinations thereof. A membrane domain can be located at any position in the polypeptide, including the N-terminal, C-terminal, or middle of the polypeptide. A membrane domain can be associated with permanent or temporary attachment of a polypeptide to a membrane. In some embodiments, a recombinant vector comprises the expression cassette. Recombinant vectors include, but are not limited to, plasmids, phages, and viruses. In some embodiments, the recombinant vector is a linearized vector. In some embodiments, the recombinant vector is an expression vector. As used herein, the phrase "expression vector" refers to a vector that is suitable for production of an encoded product (e.g., a sucrase, a sucrose transporter, or a combination thereof). In some embodiments, a polynucleotide sequence encoding the sucrase, sucrose transporter, or combination thereof is inserted into the recombinant vector to produce a recombinant nucleic acid molecule. In some embodiments, the polynucleotide sequence encoding the sucrase, sucrose transporter, or combination thereof is inserted into the vector in a manner that operatively links the nucleic acid sequence to regulatory sequences in the vector, which enables the transcription and translation of the nucleic acid sequence within the recombinant microorganism. In some embodiments, a selectable marker enables the selection of a recombinant microorganism into which a recombinant nucleic acid molecule of the present invention has successfully been introduced. Selectable markers include but are not limited to the selectable markers described in U.S. Publ. No. 2010/0233760 and U.S. Patent No. 7,001,772, such as *Schizochytrium* acetolactate synthase or bacterial ble. In some embodiments, the selection marker is an auxotrophic marker, a dominant selection marker (such as, for example, an enzyme that degrades antibiotic activity), or another protein involved in transformation selection.

In some embodiments, the thraustochytrid cell is genetically modified to introduce or delete genes involved in biosynthetic pathways associated with the transport and/or synthesis of carbohydrates, including those involved in glycosylation. For example, the thraustochytrid cell can be modified by deleting endogenous glycosylation genes and/or inserting human or animal glycosylation genes to allow for glycosylation patterns that more closely resemble those of humans. Modification of glycosylation in yeast is exemplified in, for example, U.S. Patent No. 7,029,872 and U.S. Publ. Nos. 2004/0171826, 2004/0230042, 2006/0257399, 2006/0029604, and 2006/0040353. In some embodiments, the thraustochytrid cell includes a cell in which RNA viral elements are employed to increase or regulate gene expression.

A polynucleotide sequence of any of the methods of the invention encodes a heterologous polypeptide comprising an amino acid sequence that is at least 90%, at least 91%, at least 92%, at least 93%, at least 94%, at least 95%, at least 96%, at least 97%, at least 98%, at least 99%, or that is identical to the amino acid sequence of a polypeptide associated with an invertase. In some embodiments, a polynucleotide sequence of any of the methods of the invention encodes a heterologous polypeptide comprising an amino acid sequence that is at least 90%, at least 91%, at least 92%, at least 93%, at least 94%, at least 95%, at least 96%, at least 97%, at least 98%, at least 99%, or that is identical to: the amino acid sequence of the invertase SUC2 from *Saccharomyces cerevisiae;* the invertase SacC from *Zymomonas*; the invertase INV1 from *Pichia;* the invertase INV from *Debaryomyces*; an invertase from other fungal, protist, algal, plant, or eukaryotic sources; or an invertase from a prokaryotic source such as coryneform bacteria or *Escherichia coli.* In some embodiments, a polynucleotide sequence of any of the methods of the invention encodes a polypeptide comprising an amino acid sequence that is at least 90%, at least 91%, at least 92%, at least 93%, at least 94%, at least 95%, at least 96%, at least 97%, at least 98%, at least 99%, or that is identical to the amino acid sequence encoded by Accession No. L47346. In some embodiments, a polynucleotide sequence of any of the methods of the invention encodes a polypeptide comprising an amino acid sequence that is at least 90%, at least 91%, at least 92%, at least 93%, at least 94%, at least 95%, at least 96%, at least 97%, at least 98%, at least 99%, or that is identical to the amino acid sequence encoded by SEQ ID NO:1. In some embodiments, a polynucleotide sequence of any of the methods of the invention is at least 90%, at least 91%, at least 92%, at least 93%, at least 94%, at least 95%, at least 96%, at least 97%, at least 98%, at least 99%, or that is identical to: the polynucleotide sequence of Accession No. L47346
or the polynucleotide sequence of SEQ ID NO:1.

### Thraustochytrid Cultures, Biomasses, and Microbial Oils

The present invention is also directed to a thraustochytrid cell culture comprising any of the thraustochytrid cells of the invention as described herein and a cell culture medium comprising sucrose as a carbon source.

Described herein is a method of producing a thraustochytrid cell culture, comprising transforming a thraustochytrid cell with a nucleic acid molecule comprising a polynucleotide sequence encoding a polypeptide associated with sucrose metabolism as described herein, selecting the transformed thraustochytrid cell, and growing the transformed thraustochytrid cell in a culture medium comprising sucrose as a carbon source, wherein a thraustochytrid cell culture is produced. Described herein is a thraustochytrid cell culture produced by the method.

In some embodiments, sucrose is the primary carbon source in the cell culture medium. In some embodiments, sucrose is the only carbon source in the cell culture medium. Sucrose sources include but are not limited to sugar cane, sugar beets, and other plants. The composition of the purified or partially purified sucrose can be predominantly sucrose, i.e., purified/refined sucrose, crude sucrose extracts, or a mixture of sucrose, glucose, and fructose (e.g., molasses).

In some embodiments, the culture medium comprises molasses, a syrup, or juice from any sucrose-producing vegetable (e.g., sugar cane juice or sugar beet juice), or combinations thereof. In some embodiments, the culture medium comprises a cellulose-containing feedstock, such as sugar cane bagasse. In some embodiments, the cell culture medium comprises a sucrase. Described herein, the thraustochytrid cell secretes a heterologous sucrase into the culture medium.

Culture conditions for thraustochytrid cells include, but are not limited to, effective media, bioreactor, temperature, pH, and oxygen conditions that permit protein production and/or recombination. An effective medium refers to any medium in which a thraustochytrid cell is typically cultured. Such medium typically comprises an aqueous medium having assimilable carbon, nitrogen, and phosphate sources, as well as appropriate salts, minerals, metals, and other nutrients, such as vitamins. Nitrogen sources include, but are not limited to, peptone, yeast extract, polypeptone, malt extract, meat extract, casamino acid, corn steep liquor, organic nitrogen sources, sodium glutamate, urea, inorganic nitrogen sources, ammonium acetate, ammonium sulfate, ammonium chloride, ammonium nitrate, and sodium sulfate. Non-limiting culture conditions suitable for thraustochytrids are described, for example, in U.S. Patent No. 5,340,742. Various fermentation parameters for inoculating, growing, and recovering microflora are also described, for example, in U.S. Patent No. 5,130,242. Liquid or solid media can contain natural or artificial sea water. Thraustochytrid cells of the present invention can be cultured in fermentation bioreactors, shake flasks, test tubes, microtiter dishes, and petri plates.

The fermentation volume can be any volume used for the growth of thraustochytrids, including commercial and industrial volumes. In some embodiments, the fermentation volume (volume of culture) is at least 2 L, at least 10 L, at least 50 L, at least 100 L, at least 200 L, at least 500 L, at least 1000 L, at least 10,000 L, at least 20,000 L, at least 50,000 L, at least 100,000 L, at least 150,000 L, at least 200,000 L, or at least 250,000 L, at least 300,000 L, at least 350,000 L, at least 400,000 L, or at least 500,000 L. In some embodiments, the fermentation volume is 2 L to 2,000,000 L, 2 L to 1,000,000 L, 2 L to 500,000 L, 2 L to 200,000 L, 2 L to 100,000 L, 2 L to 50,000 L, 2 L to 25,000 L, 2 L to 20,000 L, 2 L to 15,000 L, 2 L to 10,000 L, 2 L to 5,000 L, 2 L to 1,000 L, 2 L to 500 L, or 2 L to 100 L.

The present invention is also directed to a method of producing a thraustochytrid biomass comprising growing a thraustochytrid cell of the invention in a culture medium comprising sucrose as a carbon source, and harvesting a biomass from the culture medium. A thraustochytrid biomass is a harvested cellular biomass obtained by any conventional method for the isolation of a thraustochytrid biomass, such as described in U.S. Pat. No. 5,130,242 and U.S. Publ. No. 2002/0001833. A harvested biomass can contain thraustochytrid cells as well as thraustochytrid cellular fragments.

The present invention is also directed to a method of producing a microbial oil comprising growing a thraustochytrid cell of the invention in a culture medium comprising sucrose as a carbon source to produce a biomass, and extracting an oil from the biomass. The oil can be extracted from a freshly harvested biomass or can be extracted from a previously harvested biomass that has been stored under conditions that prevent spoilage. Known methods can be used to culture a thraustochytrid of the invention, to isolate a biomass from the culture, to extract a microbial oil from the biomass, and to analyze the fatty acid profile of oils extracted from the biomass. *See,* e.g., U.S. Pat. No. 5,130,242.

A microbial oil can be any oil derived from any thraustochytrid, including, for example: a crude oil extracted from the biomass of a thraustochytrid without further processing; a refined oil that is obtained by treating a crude oil with further processing such as refining, bleaching, and/or deodorizing; a diluted oil obtained by diluting a crude or refined oil; or an enriched oil that is obtained, for example, by treating a crude or refined oil with further methods of purification to increase the concentration of a fatty acid in the oil.

In some embodiments, the crude oil can be isolated from a thraustochytrid using standard techniques, without being subjected to further refinement or purification. For example, the crude oil can be isolated using solvent extraction, such as, but not limited to, hexane extraction or isopropanol extraction. In some embodiments, the crude oil can be isolated using physical and/or mechanical extraction methods such as, but not limited to, extraction through the use of a homogenizer or by pressing.

In some embodiments, the crude oil can be subjected to further processing, such as refining, desolventization, deodorization, winterization, chill filtration, and/or bleaching. Such "processed" oils include microbial oils that have been subjected to solvent extraction and one or more further processing. In some embodiments, oils are minimally processed. "Minimally processed" oils include microbial oils that have been subjected to solvent extraction and filtration. In certain embodiments, minimally processed oils are further subjected to winterization.

In some embodiments, a method similar to the FRIOLEX® process (Westfalia Separator Industry GmbH, Germany) is used to extract the microbial oils produced by the thraustochytrids. The FRIOLEX® process is a water-based physical oil extraction process, whereby raw material containing oil can be used directly for extracting oil without using any conventional solvent extraction methods. In this process, a watersoluble organic solvent can be used as a process aid and the oil is separated from the raw material broth by density separation using gravity or centrifugal forces. WO 96/05278 discloses such extraction methods.

After the oil has been extracted, the oil can be recovered or separated from non-lipid components by any suitable means known in the art. In some embodiments, low-cost physical and/or mechanical techniques are used to separate the lipid-containing compositions from non-lipid compositions. For example, if multiple phases or fractions are created by the extraction method used to extract the oil, where one or more phases or fractions contain lipids, a method for recovering the lipid-containing phases or fractions can involve physically removing the lipid-containing phases or fractions from the non-lipid phases or fractions, or vice versa. In some embodiments, a FRIOLEX® type method is used to extract the lipids produced by the microorganisms, and the lipid-rich light phase is then physically separated from the protein-rich heavy phase (such as by skimming off the lipid-rich phase that is on top of the protein-rich heavy phase after density separation).

The microbial oils produced by thraustochytrids of the present invention can be recovered from autolysis or induced lysis by exposing thraustochytrid cells to a condition including, but not limited to, a certain pH, a certain temperature, the presence of an enzyme, the presence of a detergent, physical disruptions, or combinations thereof. In some embodiments, lysis or autolysis of the thraustochytrid cells is performed by the use of mechanical forces. In further embodiments of the present invention, the lysis or autolysis of thraustochytrid cells is followed by mechanical separation of the lipids from the non-lipid compositions.

Suitable enzymes that can be used to induce lysis of the thraustochytrid cells include, but are not limited to, commercially available enzymes or enzyme mixtures such as Proteinase K or ALCALASE®. In some embodiments, the oil-producing thraustochytrids undergo induced lysis in the presence of a detergent such as ionic (cationic or anionic) detergents, nonionic detergents, zwitterionic detergents, or combinations thereof. In further embodiments of the present invention, physical disruption methods such as mechanical grinding, liquid homogenization, use of high frequency sound waves in sonication, freeze/thaw cycles methods, pressing, extruding, or milling can be used to induce lysis of the oil-producing thraustochytrids. The extraction of the oils can take place in the fermentors at the end of the fermentation by in-tank lysis of the thraustochytrid cells.

In some embodiments, the cell cultures, biomasses, and microbial oils produced from the thraustochytrid cells of the invention grown on sucrose have the same or substantially similar characteristics (e.g., cell densities, dry cell weights, fatty acid productivities, and fatty acid profiles) associated with cultures, biomasses, and microbial oils produced from the corresponding untransformed thraustochytrid cells grown on glucose and/or fructose. In some embodiments, the dry cell weight of a biomass produced from a thraustochytrid culture of the invention is higher after growth on sucrose than the dry cell weight obtained under identical conditions from growth of a culture of the corresponding untransformed thraustochytrid cells on glucose and/or fructose. In some embodiments, the total amount of fatty acids as a percentage of the dry cell weight of the biomass is higher after growth of a thraustochytrid of the invention on sucrose than the amount obtained under identical conditions from growth of a corresponding untransformed thraustochytrid cell on glucose and/or fructose.

### Methods of Producing Compositions

The present invention is also directed to a method of preparing a food product, cosmetic, industrial composition, or pharmaceutical composition for animals or humans, comprising growing a thraustochytrid cell of the invention in a culture medium comprising sucrose as a carbon source to produce a biomass, harvesting the biomass, and preparing the food product, cosmetic, industrial composition, or pharmaceutical composition for animals or humans from the biomass.

Thraustochytrid biomasses can be dried prior to use in a composition by methods including, but not limited to, freeze drying, air drying, spray drying, tunnel drying, vacuum drying (lyophilization), or a similar process. Alternatively, a harvested and washed biomass can be used directly in a composition without drying. *See,* e.g., U.S. Pat. Nos. 5,130,242 and 6,812,009.

In some embodiments, the method of preparing a food product, cosmetic, industrial composition, or pharmaceutical composition for animals or humans, further comprises extracting an oil from the biomass. Microbial oils can be used as starting material to more efficiently produce a product enriched in a fatty acid such as DHA or EPA. For example, the microbial oils can be subjected to various purification techniques known in the art, such as distillation or urea adduction, to produce a higher potency product with higher concentrations of DHA, EPA, or another fatty acid. The microbial oils can also be used in chemical reactions to produce compounds derived from fatty acids in the oils, such as esters and salts of DHA, EPA, or another fatty acid.

Any thraustochytrid that has been transformed as described herein to grow on sucrose can be selected for preparing any of the described compositions based on a desirable attribute of the thraustochytrid, such as but not limited to the cell density of a cell culture of the thraustochytrid, the percentages and types of fatty acids associated with the dry cell of a biomass of the thraustochytrid, the fatty acid profile associated with the biomass and/or the microbial oil of the thraustochytrid, or a combination thereof. In some embodiments, the thraustochytrid biomass or microbial oil comprises a greater percentage of polyunsaturated fatty acids than monounsaturated fatty acids. In some embodiments, the thraustochytrid biomass or microbial oil comprises a greater percentage of omega-3 fatty acids than omega-6 fatty acids. In some embodiments, the thraustochytrid biomass or microbial oil comprises a greater percentage of DHA than other omega-3 fatty acids. In some embodiments, the thraustochytrid biomass comprises a greater percentage of DHA than other polyunsaturated fatty acids. In some embodiments, the thraustochytrid biomass or microbial oil comprises a greater percentage of EPA than other omega-3 fatty acids. In some embodiments, the thraustochytrid biomass or microbial oil comprises a greater percentage of EPA than other polyunsaturated fatty acids.

A composition can include one or more excipients. As used herein, "excipient" refers to a component, or mixture of components, that is used in a composition to give desirable characteristics to the composition, including foods as well as pharmaceutical, cosmetic, and industrial compositions. An excipient can be described as a "pharmaceutically acceptable" excipient when added to a pharmaceutical composition, meaning that the excipient is a compound, material, composition, salt, and/or dosage form which is, within the scope of sound medical judgment, suitable for contact with the tissues of human beings and animals without excessive toxicity, irritation, allergic response, or other problematic complications over the desired duration of contact commensurate with a reasonable benefit/risk ratio. In some embodiments, the term "pharmaceutically acceptable" means approved by a regulatory agency of the Federal or a state government or listed in the U.S. Pharmacopeia or other generally recognized international pharmacopeia for use in animals, and more particularly in humans. Various excipients can be used. In some embodiments, the excipient can be, but is not limited to, an alkaline agent, a stabilizer, an antioxidant, an adhesion agent, a separating agent, a coating agent, an exterior phase component, a controlled-release component, a solvent, a surfactant, a humectant, a buffering agent, a filler, an emollient, or combinations thereof. Excipients in addition to those discussed herein can include excipients listed in, though not limited to, Remington: The Science and Practice of Pharmacy, 21st ed. (2005).

In some embodiments, the composition is a food product. A food product is any food for animal or human consumption, and includes both solid and liquid compositions. A food product can be an additive to animal or human foods. Foods include, but are not limited to, common foods; liquid products, including milks, beverages, therapeutic drinks, and nutritional drinks; functional foods; supplements; nutraceuticals; infant formulas, including formulas for pre-mature infants; foods for pregnant or nursing women; foods for adults; geriatric foods; and animal foods.

In some embodiments, a thraustochytrid biomass or microbial oil can be used directly as or included as an additive within one or more of: an oil, shortening, spread, other fatty ingredient, beverage, sauce, dairy-based or soy-based food (such as milk, yogurt, cheese and ice-cream), a baked good, a nutritional product, e.g., as a nutritional supplement (in capsule or tablet form), a vitamin supplement, a diet supplement, a powdered drink, a finished or semi-finished powdered food product, and combinations thereof.

A partial list of food compositions that can include a microbial oil includes, but is not limited to, soya based products (milks, ice creams, yogurts, drinks, creams, spreads, whiteners); soups and soup mixes; doughs, batters, and baked food items including, for example, fine bakery wares, breakfast cereals, cakes, cheesecakes, pies, cupcakes, cookies, bars, breads, rolls, biscuits, muffins, pastries, scones, croutons, crackers, sweet goods, snack cakes, pies, granola/snack bars, and toaster pastries; candy; hard confectionery; chocolate and other confectionery; chewing gum; liquid food products, for example milks, energy drinks, infant formula, carbonated drinks, teas, liquid meals, fruit juices, fruit-based drinks, vegetable-based drinks; multivitamin syrups, meal replacers, medicinal foods, and syrups; powdered beverage mixes; pasta; processed fish products; processed meat products; processed poultry products; gravies and sauces; condiments (ketchup, mayonnaise, etc.); vegetable oil-based spreads; dairy products; yogurt; butters; frozen dairy products; ice creams; frozen desserts; frozen yogurts; semi-solid food products such as baby food; puddings and gelatin desserts; processed and unprocessed cheese; pancake mixes; food bars including energy bars; waffle mixes; salad dressings; replacement egg mixes; nut and nut-based spreads; salted snacks such as potato chips and other chips or crisps, corn chips, tortilla chips, extruded snacks, popcorn, pretzels, potato crisps, and nuts; specialty snacks such as dips, dried fruit snacks, meat snacks, pork rinds, health food bars and rice/corn cakes.

In some embodiments, a microbial oil can be used to supplement infant formula. Infant formula can be supplemented with a microbial oil alone or in combination with a physically refined oil derived from an arachidonic acid (ARA)-producing microorganism. An ARA-producing microorganism, for example, is *Mortierella alpina* or *Mortierella* sect. *schmuckeri.* Alternatively, infant formulas can be supplemented with a microbial oil in combination with an oil rich in ARA, including ARASCO® (Martek Biosciences, Columbia, MD).

In some embodiments, the composition is an animal feed. An "animal" means any non-human organism belonging to the kingdom Animalia, and includes, without limitation, aquatic animals and terrestrial animals. The term "animal feed" or "animal food" refers to any food intended for non-human animals, whether for fish; commercial fish; ornamental fish; fish larvae; bivalves; mollusks; crustaceans; shellfish; shrimp; larval shrimp; artemia; rotifers; brine shrimp; filter feeders; amphibians; reptiles; mammals; domestic animals; farm animals; zoo animals; sport animals; breeding stock; racing animals; show animals; heirloom animals; rare or endangered animals; companion animals; pet animals such as dogs, cats, guinea pigs, rabbits, rats, mice, or horses; primates such as monkeys (e.g., cebus, rhesus, African green, patas, cynomolgus, and cercopithecus), apes, orangutans, baboons, gibbons, and chimpanzees; canids such as dogs and wolves; felids such as cats, lions, and tigers; equids such as horses, donkeys, and zebras; food animals such as cows, cattle, pigs, and sheep; ungulates such as deer and giraffes; rodents such as mice, rats, hamsters and guinea pigs; and so on. An animal feed includes, but is not limited to, an aquaculture feed, a domestic animal feed including pet feed, a zoological animal feed, a work animal feed, a livestock feed, or a combination thereof.

In some embodiments, the composition is a feed or feed supplement for any animal whose meat or products are consumed by humans, such as any animal from which meat, eggs, or milk is derived for human consumption. When fed to such animals, nutrients such as LC-PUFAs can be incorporated into the flesh, milk, eggs or other products of such animals to increase their content of these nutrients.

In some embodiments, the composition is a spray-dried material that can be crumbled to form particles of an appropriate size for consumption by zooplankton, artemia, rotifers, and filter feeders. In some embodiments, the zooplankton, artemia, or rotifers fed by the composition are in turn fed to fish larvae, fish, shellfish, bivalves, or crustaceans.

In some embodiments, the composition is a pharmaceutical composition. Suitable pharmaceutical compositions include, but are not limited to, an anti-inflammatory composition, a drug for treatment of coronary heart disease, a drug for treatment of arteriosclerosis, a chemotherapeutic agent, an active excipient, an osteoporosis drug, an anti-depressant, an anti-convulsant, an anti-Helicobacter pylori drug, a drug for treatment of neurodegenerative disease, a drug for treatment of degenerative liver disease, an antibiotic, a cholesterol lowering composition, and a triglyceride lowering composition. In some embodiments, the composition is a medical food. A medical food includes a food that is in a composition to be consumed or administered externally under the supervision of a physician and that is intended for the specific dietary management of a condition, for which distinctive nutritional requirements, based on recognized scientific principles, are established by medical evaluation.

In some embodiments, the microbial oil can be formulated in a dosage form. Dosage forms can include, but are not limited to, tablets, capsules, cachets, pellets, pills, powders and granules, and parenteral dosage forms, which include, but are not limited to, solutions, suspensions, emulsions, and dry powders comprising an effective amount of the microbial oil. It is also known in the art that such formulations can also contain pharmaceutically acceptable diluents, fillers, disintegrants, binders, lubricants, surfactants, hydrophobic vehicles, water soluble vehicles, emulsifiers, buffers, humectants, moisturizers, solubilizers, preservatives, and the like. Administration forms can include, but are not limited to, tablets, dragees, capsules, caplets, and pills, which contain the microbial oil and one or more suitable pharmaceutically acceptable carriers.

For oral administration, the microbial oil can be combined with pharmaceutically acceptable carriers well known in the art. Such carriers enable the microbial oils to be formulated as tablets, pills, dragees, capsules, liquids, gels, syrups, slurries, suspensions, and the like, for oral ingestion by a subject to be treated. In some embodiments, the dosage form is a tablet, pill or caplet. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl cellulose, sodium carboxymethyl cellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate. Pharmaceutical preparations that can be used orally include, but are not limited to, push-fit capsules made of gelatin, as well as soft, sealed capsules made of gelatin and a plasticizer, such as glycerol or sorbitol.

In some embodiments, the composition is a cosmetic. Cosmetics include, but are not limited to, emulsions, creams, lotions, masks, soaps, shampoos, washes, facial creams, conditioners, make-ups, bath agents, and dispersion liquids. Cosmetic agents can be medicinal or non-medicinal.

In some embodiments, the composition is an industrial composition. In some embodiments, the composition is a starting material for one or more manufactures. A manufacture includes, but is not limited to, a polymer; a photographic photosensitive material; a detergent; an industrial oil; or an industrial detergent. For example, U.S. Patent No. 7,259,006 describes use of DHA-containing fat and oil for production of behenic acid and production of photographic sensitive materials using behenic acid.

### Methods of Producing Biofuels

Described herein is a method for producing a biofuel from a recombinant thraustochytrid of the invention.

As used herein, "biofuel" refers to any fuel that is produced from or using a biomass or a microbial oil of a thraustochytrid of the invention, including, but not limited to, a biodiesel, a renewable diesel, a co-processed renewable diesel, a jet biofuel, a heating oil, and a fuel additive. Any method known in the art can be used to generate a biofuel from the biomasses and microbial oils of the thraustochytrids of the present invention, including but not limited to any methods described in WO 2005/035693, U.S. Publ. No. 2005/0112735, 2007/027633, WO 2006/127512, U.S. Publ. No. 2007/0099278, U.S. Publ. No. 2007/0089356, WO 2008/067605, U.S. Publ. No. 2009/0035842, and U.S. Publ. No. 2009/0064567. Any of the biofuels described herein can be blended with a fossil fuel. For example, a biodiesel can be blended with a fossil diesel at ratios of 1%-99%. Described herein, a microbial
oil of a thraustochytrid of the invention is used as a starting point for production of a biofuel even though it has not been subjected to conventional processing. Examples of conventional processing that can be avoided include refining (e.g., physical refining, silica refining or caustic refining), desolventization, deodorization, winterization, chill filtration, and/or bleaching. Thus, described herein, the oils have not been subjected to refining, desolventization, deodorization, winterization, chill filtration, bleaching, or a combination thereof.

Described herein, the method of producing a biofuel comprises growing a thraustochytrid of the invention in a culture medium comprising sucrose as a carbon source to produce a biomass, extracting an oil from the biomass, and producing a biofuel by transesterifying the oil, cracking the oil, processing the oil by thermal depolymerization, adding the oil to a traditional petroleum refining process, or a combination thereof.

Described herein, the biofuel is produced by transesterifying the oil. In some embodiments, the biofuel is a biodiesel. Various forms of biodiesel are known and include, but are not limited to, biodiesels described in WO 07/061903, U.S. Pat. No. 7,172,635, EP Pat. No. 1 227 143, WO 02/38709, WO 02/38707, and U.S. Publ. No. 2007/0113467. Transesterification of triglycerides in the oil produces long chain fatty acid esters, i.e., alkyl esters, and can be performed by any method known in the art for the production of biofuels. Described herein, the alkyl ether is a methyl ester or an ethyl ester. Described herein, the extracting the oil from the thraustochytrid biomass and transesterifying the oil can be performed simultaneously. Described herein, the extracting the oil from the thraustochytrid biomass and transesterifying the oil are performed separately. *See,* e.g., U.S. Publ. No. 2009/0064567.

Described herein, transesterification is performed using a lower alkyl alcohol and an acid or base catalyst. Alcohols suitable for use in the present invention include any lower alkyl alcohol containing from 1 to 6 carbon atoms (i.e., a C₁₋₆ alkyl alcohol, such as methyl, ethyl, isopropyl, butyl, pentyl, hexyl alcohols and isomers thereof). Described herein, the alcohol comprises from 5 wt. % to 70 wt. %, 5 wt. % to 60 wt. %, 5 wt. % to 50 wt. %, 7 wt. % to 40 wt. %, 9 wt. % to 30 wt. %, or 10 wt. % to 25 wt. % of the mixture of the oil composition, the alcohol, and a catalytic base. Described herein, the oil composition and the base can be added to either pure ethanol or pure methanol. The amount of alcohol used can vary with the solubility of the oil in the
alcohol. Any base known in the art to be suitable for use as a reactant can be used, including bases of the formula RO-M, wherein M is a monovalent cation and RO is an alkoxide of a C₁₋₆ alkyl alcohol. Examples of suitable bases include, but are not limited to, elemental sodium, sodium methoxide, sodium ethoxide, potassium methoxide, and potassium ethoxide. Described herein, the base is sodium ethoxide. Described herein, the base is added to the reaction with the oil composition and the alcohol in an amount of from 0.05 to 2.0 molar equivalents of triglycerides in the oil, 0.05 to 1.5 molar equivalents, 0.1 to 1.4 molar equivalents, 0.2 to 1.3 molar equivalents, or 0.25 to 1.2 molar equivalents. The oil comprising triglycerides, the alcohol, and the base are reacted together at a temperature and for an amount of time that allows the production of an ester from the fatty acid residues and the alcohol. Described herein, the reacting of the composition in the presence of an alcohol and a base is performed at a temperature from 20 °C to 140 °C, from 20 °C to 120 °C, from 20 °C to 110 °C, from 20 °C to 100 °C, or from 20 °C to 90 °C. Described herein, the reacting of the composition in the presence of an alcohol and a base is performed at a temperature of at least 20 °C, at least 75 °C, at least 80 °C, at least 85 °C, at least 90 °C, at least 95 °C, at least 105 °C, or at least 120 °C. Described herein, the reacting of the composition in the presence of an alcohol and a base is performed at a temperature of 20 °C, 75 °C, 80 °C, 85 °C, 90 °C, 95 °C, 105 °C, or 120 °C. Described herein, the reacting of the composition in the presence of an alcohol and a base is performed for a time from 2 hours to 36 hours, from 3 hours to 36 hours, from 4 hours to 36 hours, from 5 hours to 36 hours, or from 6 hours to 36 hours. Described herein, the reacting of the composition in the presence of an alcohol and a base is performed for 0.25, 0.5, 1, 2, 4, 5, 5.5, 6, 6.5, 7, 7.5, 8, 8.5, 10, 12, 16, 20, 24, 28, 32, or 36 hours. Described herein, the reacting of the oil composition, alcohol, and base can be conducted by refluxing the components to produce the fatty acid esters, such as PUFA esters. Described herein, the reacting of the oil composition can be carried out at a temperature that does not result in the refluxing of the reaction components. For example, carrying out the reacting the oil composition under pressures greater than atmospheric pressure can increase the boiling point of the solvents present in the reaction mixture. Under such conditions, the reaction can occur at a temperature at which the solvents would boil at atmospheric pressure, but would not result in the refluxing of the reaction components. Described herein, the reaction is conducted at a pressure from 5 pounds per square inch (psi) to 20 psi; from 7 psi to
15 psi; or from 9 psi to 12 psi. Described herein, the reaction is conducted at a pressure of 7, 8, 9, 10, 11, or 12 psi. Reactions conducted under pressure can be carried out at the reaction temperatures listed above. Described herein, reactions conducted under pressure can be carried out at least from 20 °C to 140 °C, from 20 °C to 120 °C, from 20 °C to 110 °C, from 20 °C to 100 °C, or from 20 °C to 90 °C. Described herein, reactions conducted under pressure can be carried out at least 70 °C, at least 75 °C, at least 80 °C, at least 85 °C, or at least 90 °C. Described herein, reactions conducted under pressure can be carried out at 70 °C, 75 °C, 80 °C, 85 °C, or 90 °C.

Reduced amounts of PUFA in an oil used for producing a biofuel, such as a biodiesel, can increase the energy density of the biofuel and can reduce the number of sites for potential oxidation or sulfation. Described herein, the thraustochytrid biomass or microbial oil, or triglyceride fraction thereof, comprises a greater percentage of monounsaturated fatty acids than polyunsaturated fatty acids. Described herein, the microbial oil is fractionated to remove polyunsaturated fatty acids. Described herein, the oil is hydrogenated in order to convert polyunsaturated fatty acids to monounsaturated fatty acids. To ensure that greater percentages of microalgal oil-derived biodiesel can be burned, any method of partial or total oil hydrogenation, as is routine in the manufacture of margarines, can be used. Described herein, thraustochytrids that produce low levels of PUFAs are used to produce the microbial oils. Described herein, thraustochytrids of the invention are selected that produce greater amounts of monounsaturated fatty acids than polyunsaturated fatty acids. Described herein, less than 50% of unsaturated fatty acids in the biological oil are PUFAs. Described herein, the unsaturated fatty acids in the biological oil contain less than 40%, less than 30%, less than 20%, less than 10%, or less than 5% PUFAs. Described herein, the microbial oil comprises less than 50%, less than 40%, less than 30%, less than 20%, less than 10%, or less than 5% by weight of PUFAs.

In addition to the transesterification methods described above, other techniques of reducing the viscosity of the microbial oils can also be used to produce biofuels. These techniques include, but are not limited to, the use of lipases, supercritical methanol catalysis, and the use of whole-cell systems involving cytoplasmic overexpression of lipases in a host cell followed by permeabilization of the host to allow catalysis of transesterification of triglycerides within the cytoplasm. Described herein, a thraustochytrid cell of the invention also comprises a polynucleotide sequence encoding a lipase for catalysis of transesterification of triglycerides within the cytoplasm. *See,* for example, U.S. Pat. No. 7,226,771, U.S. Publ. No. 2004/0005604, WO 03/089620, WO 05/086900, U.S. Publ. No. 2005/0108789, WO 05/032496, WO 05/108533, U.S. Pat. No. 6,982,155, WO 06/009676, WO 06/133698, WO 06/037334, WO 07/076163, WO 07/056786, and WO 06/124818.

Described herein, the biofuel is produced by cracking the oil. In some embodiments, the biofuel is a jet biofuel. "Cracking" is understood in the art to describe the reduction of the chain length of fatty acids in an oil by methods such as those used in the oil industry. Described herein, the presence of a significant amount of polyunsaturated fatty acid in the oil will provide greater flexibility and variety for the production of hydrocarbons, since the multiple sites of unsaturation in a polyunsaturated fatty acid provide multiple sites for cleavage to make hydrocarbons. For example, certain jet fuels require hydrocarbons with two to eight carbons. Polyunsaturated fatty acids can be cleaved through known processes in the art, such as cracking, to produce shorter hydrocarbons of various chain lengths.

Described herein, the biofuel is produced by thermal depolymerization. In some embodiments, the biofuel is a renewable diesel. As used herein, thermal depolymerization includes any process for the production of renewable diesel using superheated water.

Described herein, the biofuel is produced by adding the oil to a petroleum refining process during the production of diesel fuel. Described herein, the biofuel is a co-processed renewable diesel.

Having generally described this invention, a further understanding can be obtained by reference to the examples provided herein. These examples are for purposes of illustration only and are not intended to be limiting.

### EXAMPLE 1

### Construction of the pCL0076 expression vector

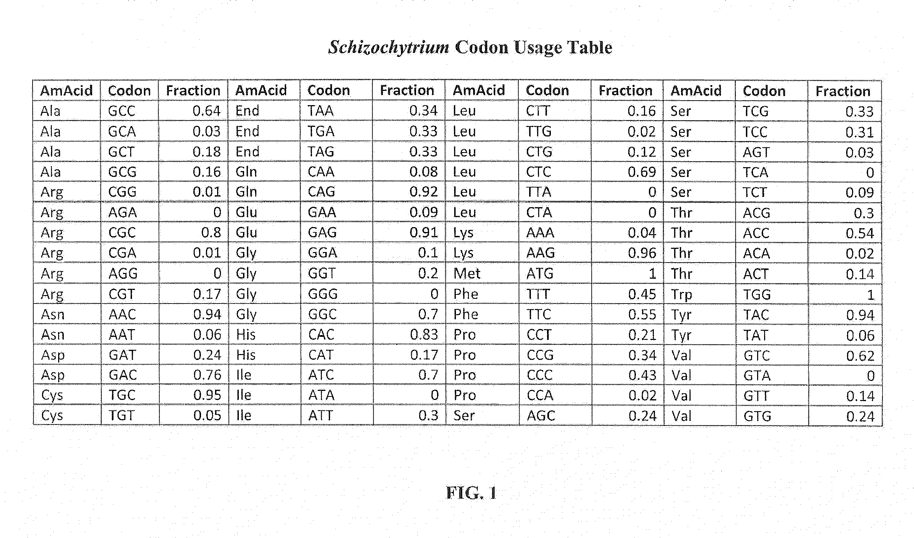

The vector pAB0018 (ATCC Accession No. PTA-9616) was digested with *Bam*HI and *Nde*I resulting in two fragments of 838 bp and 9879 bp in length. The 9879 bp fragment was fractionated by standard electrophoretic techniques in an agar gel, purified using commercial DNA purification kits, and ligated to a sequence (SEQ ID NO:1) comprising a polynucleotide sequence encoding the native secretion signal of the Sec1 protein of *Schizochytrium* sp. ATCC number 20888 followed by a synthetic sequence encoding the mature invertase protein (SUC2) of *Saccharomyces cerevisiae,* codon-optimized using the *Schizochytrium* codon usage table in **FIG. 1** (codon optimization was performed by Blue Heron Biotechnology, Bothell, WA). A fusion sequence containing the sequences encoding the *Schizochytrium* Sec1 signal peptide and the *Saccharomyces cerevisiae* SUC2 secreted invertase protein (GenBank Accession No. P00724) was inserted into the *Schizochytrium* vector pSchiz, followed by digestion with *Bam*HI and *Nde*I to yield SEQ ID NO:1.

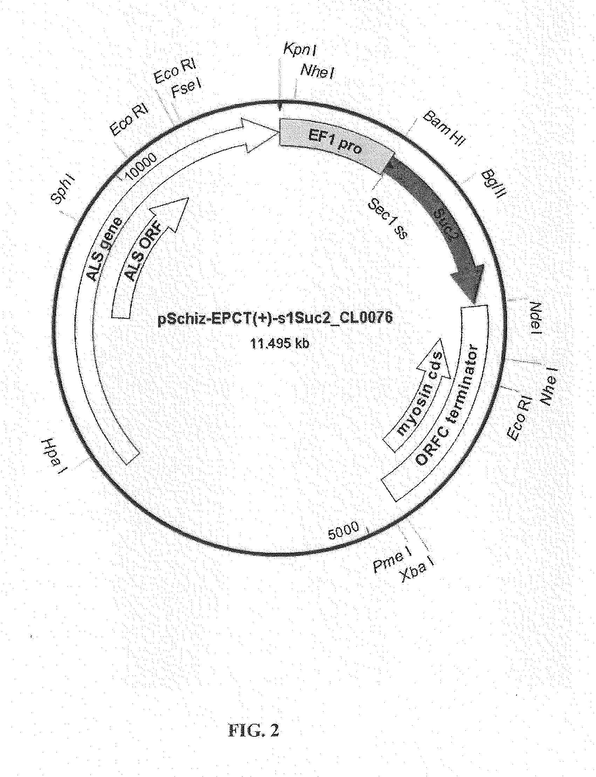

The ligation product was then used to transform a commercially supplied strain of competent DH5α *E. coli* cells (Invitrogen) using the manufacturer's protocols. Several of the resulting clones were propagated and their plasmids were extracted and purified. These plasmids were then screened by restriction digests and/or PCR to confirm that the ligation generated the expected plasmid vectors. One such plasmid vector resulting from the ligation of the 9879 bp fragment and SEQ ID NO:1 was verified by Sanger sequencing and designated pCL0076 (SEQ ID NO:2). *See* **FIG. 2****.**

### EXAMPLE 2

### Growth of Schizochytrium on Sucrose

Cultures of *Schizochytrium* sp. ATCC 20888 and a genetically modified *Schizochytrium* derivative, designated B76-32, were grown in M2B medium consisting of 10 g/L glucose, 0.8 g/L (NH₄)₂SO₄, 5 g/L Na₂SO₄, 2 g/L MgSO₄•7H₂O, 0.5 g/L KH₂PO₄, 0.5 g/L KCl, 0.1 g/L CaCl₂•2H₂O, 0.1 M MES (pH 6.0) 0.1% PB26 metals, and 0.1% PB26 Vitamins (v/v). PB26 vitamins consisted of 50 mg/mL vitamin B12, 100 µg/mL thiamine, and 100 µg/mL Ca-pantothenate. PB26 metals were adjusted to pH 4.5 and consisted of 3 g/L FeSO₄•7H₂O, 1 g/L MnCl₂•₄H₂O, 800 mg/mL ZnSO₄•7H₂O, 20 mg/mL CoCl₂•6H₂O, 10 mg/mL Na₂MoO₄•2H₂O, 600 mg/mL CuSO₄•5H₂O, and 800 mg/mL NiSO₄•6H₂O. PB26 stock solutions were filter sterilized separately and added to the broth after autoclaving. Glucose, KH₂PO₄, and CaCl₂•2H₂O were each autoclaved separately from the remainder of the broth ingredients before mixing to prevent salt precipitation and carbohydrate caramelizing. All medium ingredients were purchased from Sigma Chemical (St. Louis, MO). Strain B76-32 is a derivative of *Schizochytrium* sp. ATCC 20888 engineered according to U.S. Pat. No. 7,211,418 and U.S. Publ. Nos. 2008/0022422 and 2008/0026434.

Cultures of *Schizochytrium* sp. ATCC 20888 and B76-32 were grown to log phase and were transformed with the vector pCL0076 using electroporation with enzyme pretreatment as described below.

Electroporation with enzyme pretreatment - Cells were grown in 50 mL of M50-20 media (see U.S. Publ. No. 2008/0022422) on a shaker at 200 rpm for 2 days at 30 °C. The cells were diluted at 1:100 into M2B media (see following paragraph) and grown overnight (16-24 h), attempting to reach mid-log phase growth (OD600 of 1.5-2.5). The cells were centrifuged in a 50 mL conical tube for 5 min at about 3000 x g. The supernatant was removed and the cells were resuspended in 1 M mannitol, pH 5.5, in a suitable volume to reach a final concentration of 2 OD₆₀₀ units. 5 mL of cells were aliquoted into a 25 mL shaker flask and amended with 10 mM CaCl₂ (1.0 M stock, filter sterilized) and 0.25 mg/mL Protease XIV (10 mg/mL stock, filter sterilized; Sigma-Aldrich, St. Louis, MO). Flasks were incubated on a shaker at 30 °C and about 100 rpm for 4 h. Cells were monitored under the microscope to determine the degree of protoplasting, with single cells desired. The cells were centrifuged for 5 min at about 2500 x g in round-bottom tubes (i.e., 14 mL Falcon™ tubes, BD Biosciences, San Jose, CA). The supernatant was removed and the cells were gently resuspended with 5 mL of ice cold 10% glycerol. The cells were re-centrifuged for 5 min at about 2500 x g in round-bottom tubes. The supernatant was removed and the cells were gently resuspended with 500 µL of ice cold 10% glycerol, using wide-bore pipette tips. 90 µL of cells were aliquoted into a prechilled electro-cuvette (Gene Pulser® cuvette - 0.1 cm gap or 0.2 cm gap, Bio-Rad, Hercules, CA). One µg to 5 µg of DNA (in less than or equal to a 10 µL volume) was added to the cuvette, mixed gently with a pipette tip, and placed on ice for 5 min. Cells were electroporated at 200 ohms (resistance), 25 µF (capacitance), and either 250V (for 0.1 cm gap) or 500V (0.2 cm gap). 0.5 mL of M50-20 media was added immediately to the cuvette. The cells were then transferred to 4.5 mL of M50-20 media in a 25 mL shaker flask and incubated for 2-3 h at 30 °C and about 100 rpm on a shaker. The cells were centrifuged for 5 min at about 2500 x g in round bottom tubes. The supernatant was removed and the cell pellet was resuspended in 0.5 mL of M50-20 media. Cells were plated onto an appropriate number (2 to 5) of M2B plates with appropriate selection (if needed) and incubated at 30 °C.

Transformants were selected for growth in either M2B + SMM media or directly selected for growth on sucrose by plating onto MSFM + sucrose. For MSFM + sucrose selection, after 1-2 weeks colonies were replated with several passes onto fresh sucrose-containing media. It was determined that expression of invertase can be used as a selectable marker for thraustochytrid colonies grown on sucrose as a sole carbon source.

For the following experiments, primary transformants were selected for growth on solid M2B media containing 20 g/L agar (VWR, West Chester, PA) and 10 µg/mL SMM (Chem Service, Westchester, PA) after 2-6 days of incubation at 27 °C. All primary transformants were manually transferred to fresh M2B plates with SMM. After 1 week the colonies were transferred to MSFM and 5 g/L sucrose without SMM. After 1 week the largest colonies were transferred to fresh MSFM/sucrose media plates. Ten of the *Schizochytrium* sp. ATCC 20888 transformants growing on sucrose were selected for further characterization and were designated as 1-1, 1-3, 1-24, 3-1, 3-2, 3-5, 3-21, 4-1, 4-24, and 4-31, respectively. Nine of the B76-32 transformants growing on sucrose were selected for further characterization and were designated as B76-32 #2, #12, #19, #26, #30, #39, #42, #56, and #61.

Colonies growing on sucrose (1-1, 1-3, 1-24, 3-1, 3-2, 3-5, 3-21, 4-1, 4-24, 4-31) were removed from plates using an inoculation loop and transferred into culture tubes containing 5 mL of sucrose media and grown for 4 days at 29 °C on a shaker. 2 mL of this culture was used to inoculate 50 mL of media (MSFM or SSFM) in 250 ml flasks and grown at 29 °C on a shaker at 200 rpm.

Control flasks of the parental strain *Schizochytrium* sp. ATCC 20888 were grown the same way but using glucose containing media. Cells were harvested after 7 days. Cells were centrifuged and washed with a 50% isopropanol:distilled water mixture. The pelleted cells were freeze-dried, weighed, and a fatty acid methyl esters (FAME) analysis was performed. For FAME analysis, oils were extracted from cell cultures using standard procedures and analyzed for fatty acid composition as a percent of total fatty acid methyl esters (FAMEs). *See,* e.g., U.S. Publ. No. 2010/0239533. Growth and fat content of CL0076 transformants of *Schizochytrium* sp. ATCC 20888 or B76-32 were assayed gravimetrically and by gas chromatography of derivatized oils as previously described in U.S. Publ. No. 2008/0022422.

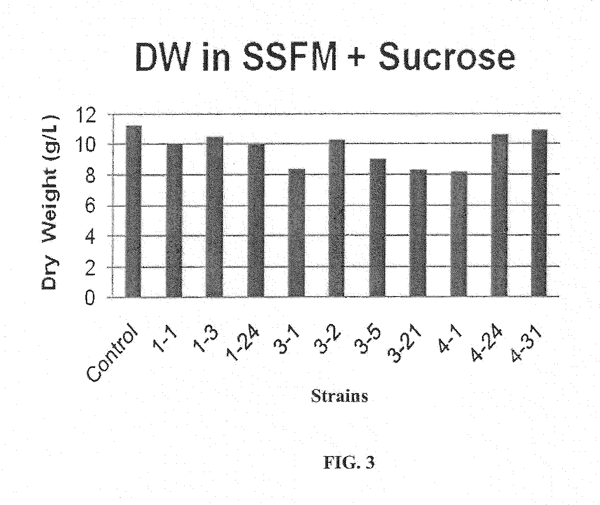

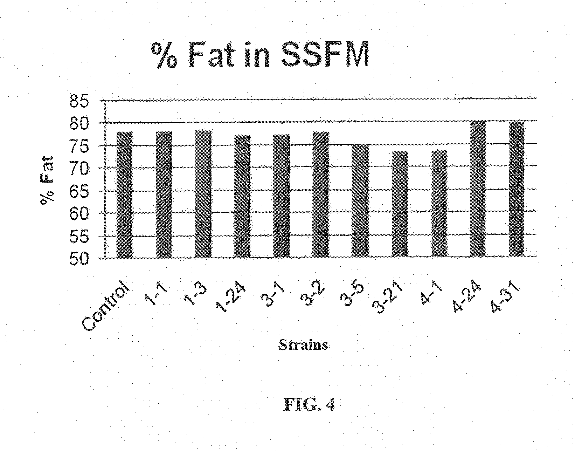

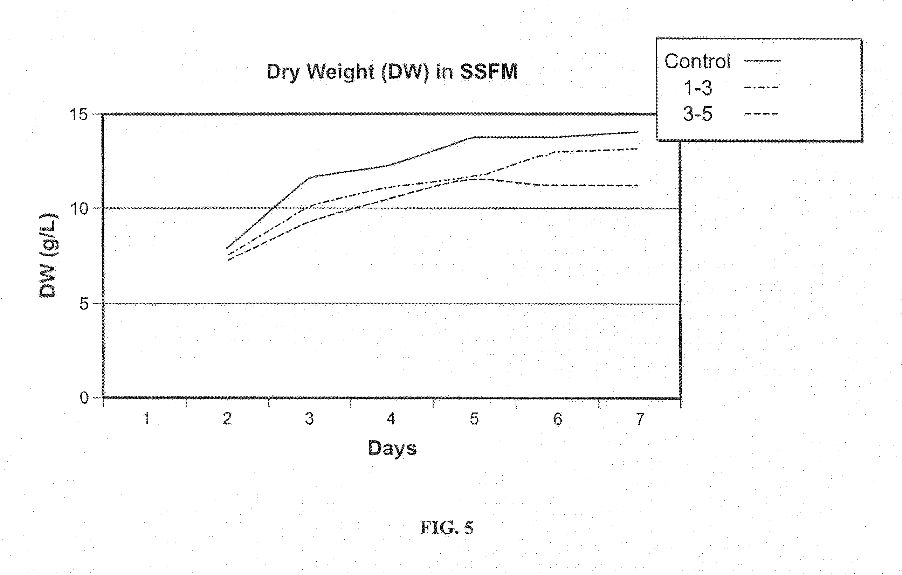

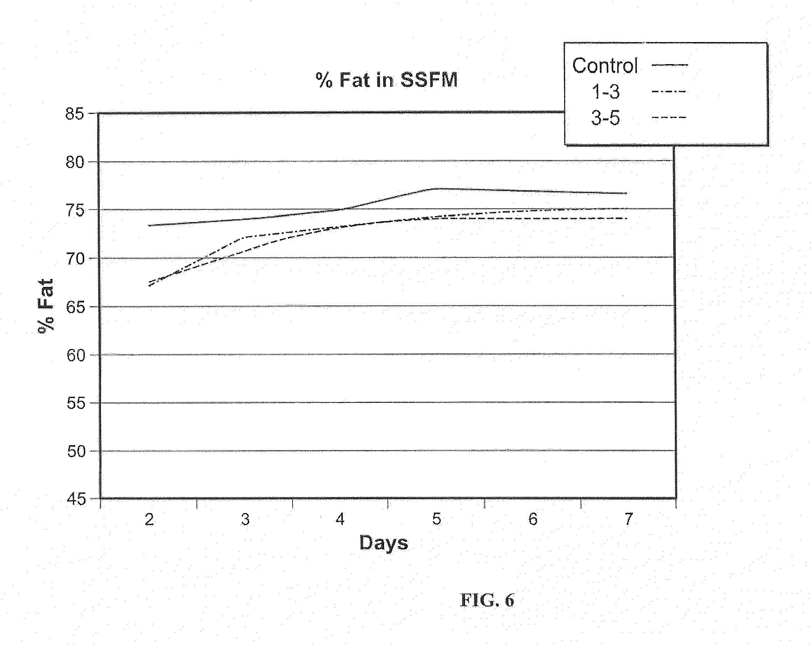

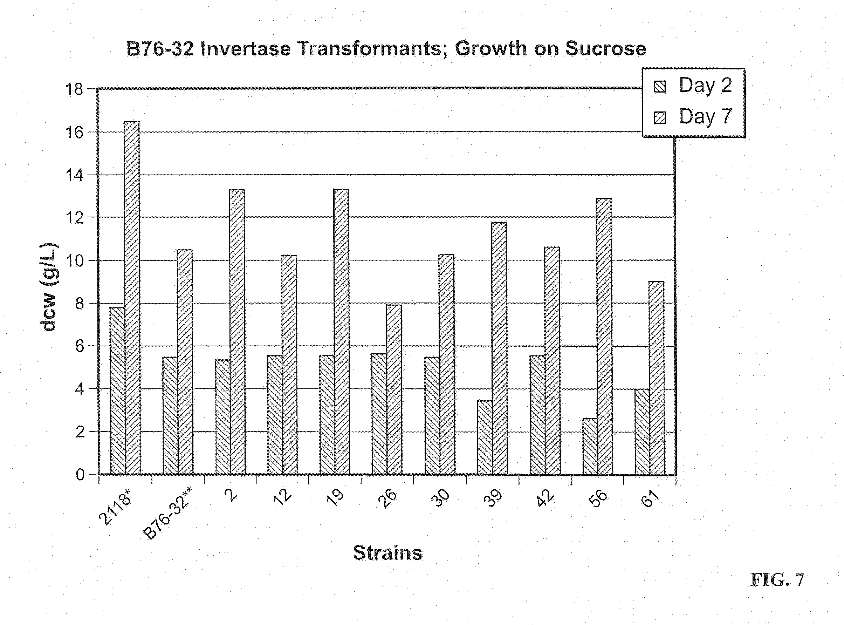

Results are shown in **Tables 5-8.** Dry weights and fat content of pellets from shake flask cultures of transformants as well as parent strains are shown in **FIGs. 3-8****.**

SSFM media: 50 g/L glucose or sucrose, 13.6 g/L Na₂SO₄, 0.7 g/L K₂SO₄, 0.36 g/L KCl, 2.3 g/L MgSO₄•7H₂O, 0.1M MES (pH 6.0), 1.2 g/L (NH₄)₂SO₄, 0.13 g/L monosodium glutamate, 0.056 g/L KH₂PO₄, and 0.2 g/L CaCl₂•2H₂O. Vitamins were added at 1 mL/L from a stock consisting of 0.16 g/L vitamin B12, 9.7 g/L thiamine, and 3.3 g/L Ca-pantothenate. Trace metals were added at 2 mL/L from a stock consisting of 1 g/L citric acid, 5.2 g/L FeSO₄•7H₂O, 1.5 g/L MnCl₂•₄H₂O, 1.5 g/L ZnSO₄•7H₂O, 0.02 g/L CoCl₂•6H₂O, 0.02 g/L Na₂MoO₄•2H₂O, 1.0 g/L CuSO₄•5H₂O, and 1.0 g/L NiSO₄•6H₂O, adjusted to pH 2.5.

Modified SFM (MSFM) media: 10 g/L glucose or sucrose, 25.0 g/L NaCl, 1.0 g/L KCl, 0.2 g/L (NH4)2SO4, 5 g/L, 5.0 g/L MgSO4•7H2O, 0.1 g/L KH₂PO₄, 0.3 g/L CaCl2•2H2O, 0.1 M HEPES (pH 7.0), 0.1% PB26 metals, and 0.1% PB26 Vitamins (v/v). Vitamins were added at 2 mL/L from a stock consisting of 0.16 g/L vitamin B12, 9.7 g/L thiamine, and 3.3 g/L Ca-pantothenate. Trace metals were added at 2 mL/L from a stock consisting of 1 g/L citric acid, 5.2 g/L FeSO4•7H2O, 1.5 g/L MnCl2•4H2O, 1.5 g/L ZnSO4•7H2O, 0.02 g/L CoCl2•6H2O, 0.02 g/L Na2MoO4•2H2O, 1.0 g/L CuSO4•5H2O, and 1.0 g/L NiSO4•6H2O, adjusted to pH 2.5.

Table 5 shows the growth and fat levels of Schizochytrium sp. ATCC 20888 grown in MSFM with glucose, fructose, sucrose, or no added carbon source.

Table 6 shows the dry weight and % fatty acid for Schizochytrium sp. ATCC 20888 grown in MSFM media with glucose (control) and Schizochytrium sp. ATCC 20888 transformed cell lines grown in MSFM media with sucrose.

Table 7 shows the dry weight and % fatty acid for Schizochytrium sp. ATCC 20888 grown in SSFM media with glucose (control) and Schizochytrium sp. ATCC 20888 transformed cell lines grown in SSFM media with sucrose.

Table 8 shows the dry weight and % fatty acid for Schizochytrium B76-32 grown in SSFM media with glucose (control) and Schizochytrium B76-32 transformed cell lines grown in SSFM media with sucrose.

**Table 5. Growth and fat levels of Schizochytrium sp. ATCC 20888 grown in MSFM with glucose, fructose, sucrose or no added carbon source.**

| | **Glucose** | **Fructose** | **Sucrose** | **No added carbon** |
|---|---|---|---|---|
| **DW (g/L)** | 2.84 | 2.65 | 0.16 | 0.11 |
| **% FA** | 66.5 | 65.3 | ND | ND |

| | | | | |
|---|---|---|---|---|
| DW= Dry Weight FA=Fatty Acids | | | | |

**Table 6. Schizochytrium sp. ATCC 20888 transformed cell lines grown in MSFM media with sucrose.**

| | **20888 control** | **1-1** | **1-3** | **3-2** | **3-5** | **3-21** | **4-1** | **4-24** | **4-31** |
|---|---|---|---|---|---|---|---|---|---|
| **DW (g/L)** | 2.94 | 2.49 | 2.79 | 2.21 | 2.60 | 2.64 | 2.44 | 3.05 | 2.24 |
| **% FA** | 70.87 | 70.79 | 72.36 | 67.97 | 69.78 | 71.05 | 68.84 | 73.85 | 73.66 |

| | | | | | | | | | |
|---|---|---|---|---|---|---|---|---|---|
| DW= Dry Weight FA=Fatty Acids | | | | | | | | | |

**Table 7. Schizochytrium sp. ATCC 20888 transformed cell lines grown in SSFM media with sucrose.**

| | **20888 control** | **1-1** | **1-3** | **1-24** | **3-1** | **3-2** | **3-5** | **3-21** | **4-1** | **4-24** | **4-31** |
|---|---|---|---|---|---|---|---|---|---|---|---|
| **DW (g/L)** | 11.24 | 10.04 | 10.51 | 9.99 | 8.40 | 10.29 | 9.03 | 8.34 | 8.16 | 10.63 | 10.92 |
| **% FA** | 78.22 | 78.20 | 76.29 | 77.10 | 77.37 | 77.71 | 74.97 | 73.44 | 73.65 | 80.05 | 79.82 |

| | | | | | | | | | | | |
|---|---|---|---|---|---|---|---|---|---|---|---|
| DW= Dry Weight FA=Fatty Acids | | | | | | | | | | | |

**Table 8. B76-32 transformed cell lines grown in SSFM media with sucrose.**

| | **B76-32 control** | **#2** | **#12** | **#19** | **#26** | **#30** | **#39** | **#42** | **#56** | **#61** |
|---|---|---|---|---|---|---|---|---|---|---|
| **7-day DW (g/L)** | 10.56 | 13.37 | 10.21 | 13.26 | 7.88 | 10.26 | 11.81 | 10.47 | 12.84 | 8.97 |
| **% FA** | 62.8 | 74.3 | 75.2 | 65.4 | 66.9 | 65.1 | 64.8 | 71.4 | 77.9 | 73.7 |

| | | | | | | | | | | |
|---|---|---|---|---|---|---|---|---|---|---|
| DW= Dry Weight FA=Fatty Acids | | | | | | | | | | |

### EXAMPLE 3

### Expression of Invertase in Schizochytrium

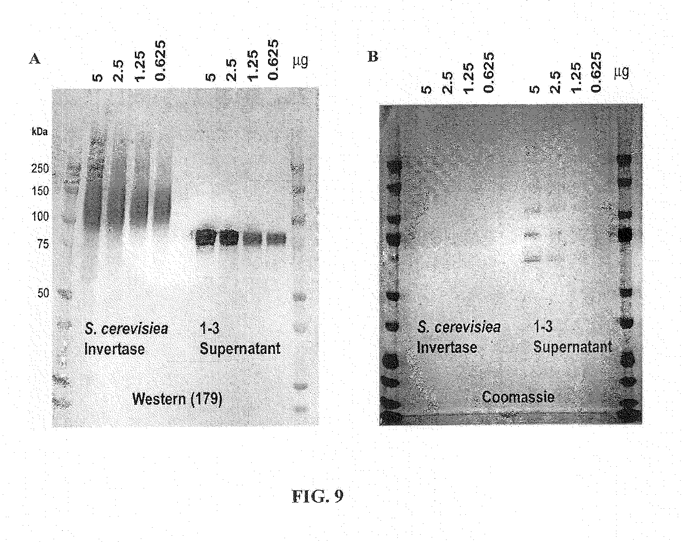

Cell-free supernatants of 50 mL shake-flask cultures of *Schizochytrium* sp. ATCC 20888 clone 1-3 transformed with pCL0076 (Example 2) grown in SSFM for 3 days (*see* U.S. Publ. No. 2008/0022422) were collected after cultures were centrifuged at 5000 x g. Culture supernatants were used either directly for SDS-PAGE or were concentrated 50 to 100-fold using commercially available concentrators equipped with permeable membranes permitting concentration of all components heavier than 10 kDa. Total protein concentration was measured by Bradford assay (Biorad). The expression of invertase was then verified by immunoblot analysis following standard immunoblotting procedure (Sambrook *et al.*)*.* Briefly, the proteins (0.625 µg to 5 µg) were separated by SDS-PAGE on a bis-tris gel (Invitrogen, Carlsbad, CA, USA). The proteins were then stained with Coomassie blue (SimplyBlue Safe Stain, Invitrogen, Carlsbad, CA, USA) or transferred onto polyvinylidene fluoride membrane and probed for the presence of invertase protein with an invertase antisera (Open Biosystems, Huntsville, AL) derived from rabbits that had been injected with a pure preparation of *Saccharomyces cerevisiae* invertase (Sigma, St. Louis, MO). The membrane was subsequently incubated with a mouse anti-rabbit secondary antibody coupled to alkaline phosphatase (Promega Corporation, Madison, WI). The membrane was then treated with 5-bromo-4-chloro-3-indoyl-phosphate/nitroblue tetrazolium solution (BCIP/NBT) according to the manufacturer's instructions (KPL, Gaithersburg, MD). An example is presented in **FIG. 9**. Anti-invertase immunoblot and corresponding Coomassie blue-stained gel are presented in panels **9A** and **9B,** respectively. Of the four major bands seen in culture supernatants of clone 1-3, only one was shown to react with anti-invertase antisera. The identity of the protein was confirmed by peptide sequence analysis.

### EXAMPLE 4

### Invertase Activity in Schizochytrium

Sucrase activity was measured by the rate of liberation of fructose and glucose from sucrose. The assay was performed by adding sucrose to fermentation broth supernatant and the glucose/fructose content was measured by HPLC.

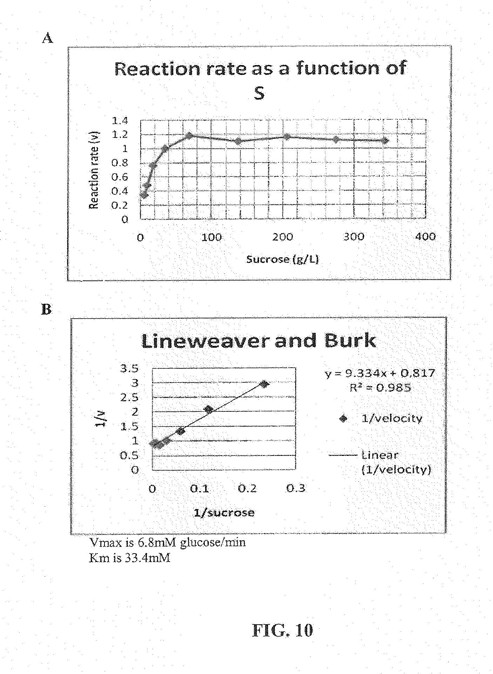

*Schizochytrium* sp. B76-32 clone #2 transformed with pCL0076 (Example 2) was grown in MSFM (with sucrose) until the OD₆₀₀ₙₘ reached about 4 in 50 mL shake flasks at 29 °C. Cells were spun down for 15 min at 4500 x g and invertase activity was measured in the supernatant. Invertase was assayed by adding 0.1 M sucrose to varying volumes of fermentation broth and adjusting the final volume to 1 mL. The reaction was incubated at 55 °C for 3 min. Termination of the reaction was done at 100 °C for 10 min, and then frozen prior to quantification of glucose, fructose, and sucrose by HPLC. HPLC was performed using a modified version of the process described in Liu et al., Food Sci. 28:293-296 (2007). Briefly, mono- and di-saccharides were separated using an HPLC with a Luna NH₂ column and detected using an RID (refractive index detector). Identification was carried out by comparing retention times to those of standards. Quantitation was by an external standard calibration. The reaction rate as a function of sucrose concentration is shown in **FIG. 10A**. The Km (33.4 mM) and Vmax (6.8 mM glucose/min) were calculated from a standard Lineweaver-Burk plot. *See* **FIG. 10B****.**

### EXAMPLE 5

### Glycosylation of Invertase in Schizochytrium

Supernatant proteins from Example 4 were separated by SDS-PAGE on a 4-12% bis-tris gel (Invitrogen). The proteins were then stained with Coomassie blue (SimplyBlue™ Safe Stain, Invitrogen, Carlsbad, CA). The stained proteins of interest were cut from the gel and slices cut into smaller pieces (∼1 mm³) and destained alternately with 40 mM Ammonium bicarbonate (AmBic) and 100% acetonitrile until the color turned clear. Destained gel was reswelled in 10 mM DTT in 40 mM Ambic at 55 °C for 1 h. The DTT solution was exchanged with 55 mM Iodoacetamide (IAM) and incubated in the dark for 45 min. Incubation was followed by washing alternately with 40 mM AmBic and 100% acetonitrile twice. Dehydrated gel was reswelled with trypsin solution (trypsin in 40 mM Ambic) on ice for 45 min initially, and protein digestion was carried out at 37 °C overnight. The supernatant was transferred into another tube. Peptides and glycopeptides were extracted from the gel in series with 20% acetonitrile in 5% formic acid, 50% acetonitrile in 5% formic acid, and then 80% acetonitrile in 5% formic acid. The sample solutions were dried and combined into one tube. Extracted tryptic digest was passed through a C18 Sep-Pak® cartridge (Waters Corporation, Milford, MA) and washed with 5% acetic acid to remove contaminants (such as salts and SDS). Peptides and glycopeptides were eluted in series with 20% isopropanol in 5% acetic acid, 40% isopropanol in 5% acetic acid, and 100% isopropanol and were dried in a speed vacuum concentrator. The dried samples were combined and then reconstituted with 50 mM sodium phosphate buffer (pH 7.5) and heated at 100 °C for 5 min to inactivate trypsin. The tryptic digest was incubated with PNGase F at 37 °C overnight to release N-glycans. After digestion, the sample was passed through a C18 Sep-Pak® cartridge and the carbohydrate fraction was eluted with 5% acetic acid and dried by lyophilization. Released N-linked oligosaccharides were permethylated based on the method of Anumula and Taylor (Anumula and Taylor, 1992) and profiled by mass spectrometry. Mass spectrometric analysis was performed following the method developed at the Complex Carbohydrates Research Center (Aoki K et al., J. Biol. Chem. 282:9127-42 (2007). Mass analysis was determined by using NSI-LTQ/MSₙ. Briefly, permethylated glycans were dissolved in 1 mM NaOH in 50% methanol and infused directly into the instrument (Finnigan™ LTQ™ Linear Ion Trap Mass Spectrometer, Thermo Electron Corporation, Waltham, MA) at a constant flow rate of 0.4 (µL/min. The MS analysis was performed in the positive ion mode. For total ion mapping, automated MS/MS analysis (at 35 collision energy), m/z range from 500 to 2000 was scanned in successive 2.8 mass unit windows that overlapped the preceding window by 2 mass units.

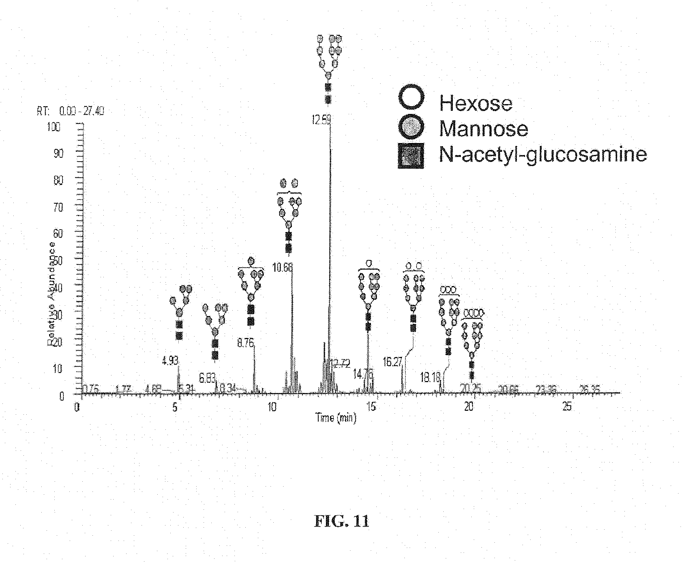

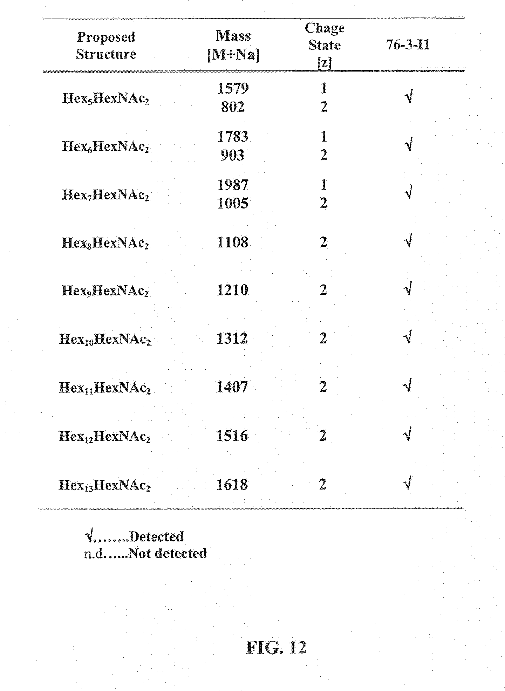

Total ion mapping was performed to examine the presence of fragment ions indicative of glycans. All MS/MS data from m/z 500 through m/z 2000 were taken and the raw data were analyzed manually. The chromatogram and table of species obtained by NSI-total ion mapping are shown in **FIG. 11** and **FIG. 12****.** This chromatogram was processed by the scan filter, neutral loss of m/z 139, which is the one of characteristic neutral loss of high-mannose type glycans. Total ion mapping revealed that this sample contains a series of high-mannose type glycans with long mannose chains.

### EXAMPLE 6

### Expression of Aspergillus niger Invertase in Schizochytrium

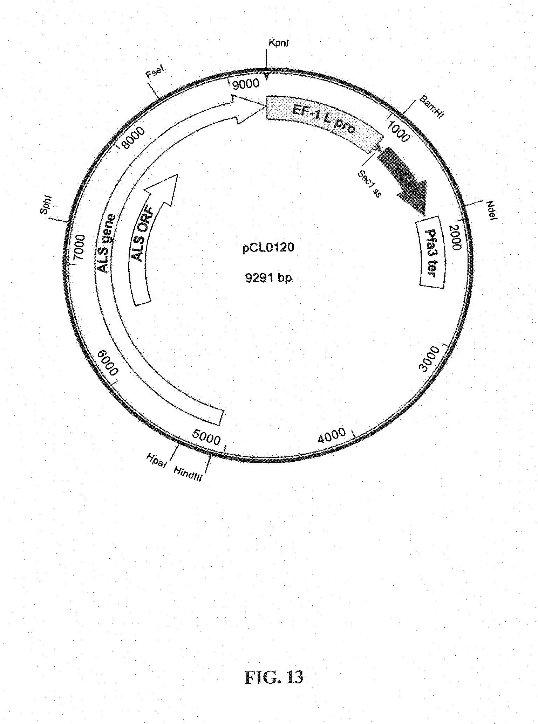

The vector pAB0018 (ATCC Accession No. PTA-9616) was digested with HindIII, treated with mung bean nuclease, purified, and then further digested with Kpnl generating four fragments of various sizes. A fragment of 2552 bp was isolated by standard electrophoretic techniques in an agar gel and purified using commercial DNA purification kits. A second digest of pAB0018 with Pmel and Kpn was then performed. A fragment of 6732 bp was isolated and purified from this digest and ligated to the 2552 bp fragment. The ligation product was then used to transform commercially supplied strains of competent DH5-α *E. coli* cells (Invitrogen) using the manufacturer's protocol. Plasmids from ampicillin-resistant clones were propagated, purified, and then screened by restriction digests or PCR to confirm that the ligation generated the expected plasmid structures. One verified plasmid was designated pCL0120. *See* **FIG. 13** and SEQ ID NO:5.

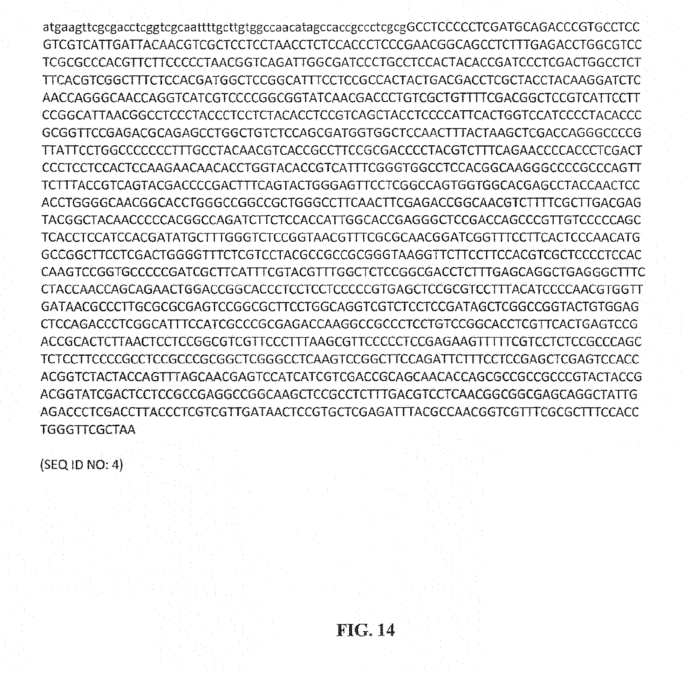

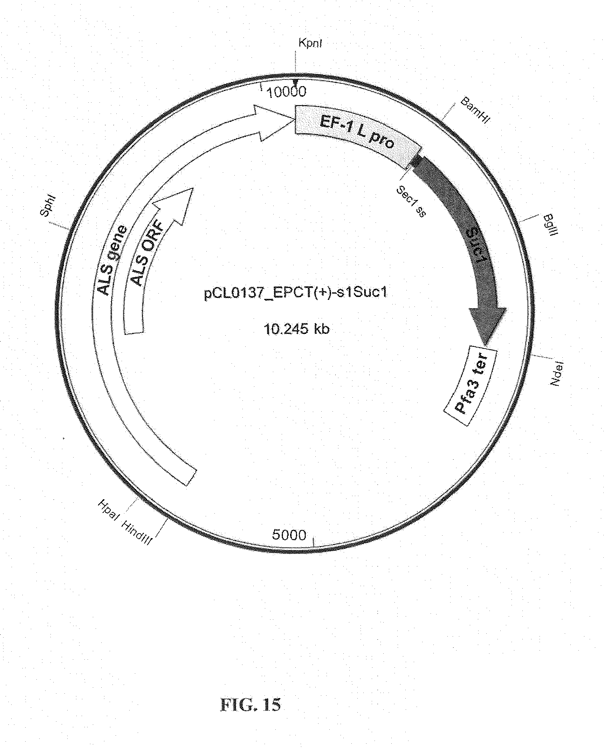

The polynucleotide sequence encoding the mature form of the Suc1 secreted invertase protein from the fungus *Aspergillus niger* (GenBank Accession No. S33920) was codon-optimized for expression in *Schizochytrium* using the *Schizochytrium* codon usage table of **FIG. 1** (codon optimization was performed by Blue Heron Biotechnology, Bothell, WA). The codon-optimized sequence was synthesized and the resulting polynucleotide sequence was fused to a polynucleotide sequence encoding the *Schizochytrium* Sec1 signal peptide ("Sec1 ss") as an N-terminal leader in place of the endogenous signal peptide. The resulting coding region of the "s1Suc1" nucleic acid sequence (SEQ ID NO:4) is shown in **FIG. 14****.** This codon-optimized slSucl polynucleotide was cloned to the vector pCL0120 using the 5' and 3' restriction sites BamHI and Ndel for insertion and ligation according to standard techniques. A plasmid map of the resulting vector, pCL0137, is shown in **FIG. 15****.** Wild-type strain *Schizochytrium* sp. ATCC 20888 was transformed with this vector and the resulting clones were selected on solid SSFM media containing SMM. SMM-resistant clones were then re-plated to SSFM solid media containing sucrose as a sole carbon source to assay for growth. Depending on the transformation experiment, between 50% and 90% of the SMM-resistant primary transformants were capable of growth on sucrose media.

### EXAMPLE 7

### Expression of Secreted Invertase, Cytoplasmic Invertase, and Sucrose Transporter in Schizochytrium

The *Saccharomyces cerevisiae* AGT1 alpha-glucoside transporter gene (GenBank Accession No. L47346) was codon-optimized for expression in *Schizochytrium* and was inserted into the vector pCL0121 (SEQ ID NO:6), comprising a ZEOCIN™ selectable marker, to produce the vector pCL0125 (SEQ ID NO:7).

The *Solanum tuberosum* (potato) SUT1 sucrose transporter gene (GenBank Accession No. X69165) was codon-optimized for expression in *Schizochytrium* and was inserted into the vector pCL0121 (SEQ ID NO:6), comprising a ZEOCIN™ selectable marker, to produce the vector pCL0126 (SEQ ID NO:8).

The polynucleotide sequence encoding the *Saccharomyces cerevisiae* cytoplasmic SUC2 invertase protein (GenBank Accession No. P00724) was codon-optimized for expression in *Schizochytrium* and was inserted into the vector pCL0122 (SEQ ID NO:9), comprising a paromomycin selectable marker, to produce the vector pCL0127 (SEQ ID NO:10).

*Schizochytrium* sp. B76-32 clone #19 transformed with pCL0076 was further transformed with either pCL0125 or pCL0126, comprising the AGT1 alpha-glucoside transporter and SUT1 sucrose transporter, respectively. Transformants were selected on antibiotics and the presence of the transporter genes was confirmed by PCR. Transformants were further transformed with pCL0127 comprising cytoplasmic SUC2, were selected on antibiotic, and were analyzed for growth on sucrose based on dry weight and FAME content as described in Example 2. Growth on sucrose and production of fatty acids were observed.

Wild type *Schizochytrium* sp. ATCC 20888 cells were transformed with either: (1) pCL0125, comprising the AGT1 alpha-glucoside transporter, in combination with pCL0127, comprising cytoplasmic SUC2; or (2) pCL0126, comprising the SUT1 sucrose transporter, in combination with pCL0127, comprising cytoplasmic SUC2. Transformants were grown and selected on sucrose. Each of the combinations of pCL0125/pCL0127 and pCL0126/pCL0127 produced transformants capable of growth on sucrose. Positive transformants were identified and frozen. At a subsequent date, the positive transformants are thawed, are further transformed with pCL0076 comprising secreted invertase, are selected on antibiotic, and are analyzed for growth levels and FAME as described in Example 2.

*Schizochytrium* sp. ATCC 20888 transformed with pCL0076 comprising the SUC2 secreted invertase (clone 1-3, Example 2) was further transformed with either: (1) pCL0125, comprising the AGT1 alpha-glucoside transporter, in combination with pCL0127, comprising cytoplasmic SUC2; or (2) pCL0126, comprising the SUT1 sucrose transporter, in combination with pCL0127, comprising cytoplasmic SUC2. Transformants were grown and selected on ZEOCIN™ and paromomycin. Positive transformants were identified and frozen. At a subsequent date, the positive transformants are thawed and are analyzed for growth on sucrose and for fatty acid methyl esters as described in Example 2.

## Claims

1. A method of producing a thraustochytrid cell culture, comprising:
(a) transforming a thraustochytrid cell with a nucleic acid molecule comprising a polynucleotide sequence encoding a heterologous invertase, wherein the invertase is bound to the plasma membrane of the thraustochytrid cell, and
(b) growing the transformed thraustochytrid cell in a culture medium comprising sucrose as a carbon source.

2. A thraustochytrid cell comprising a nucleic acid molecule comprising a polynucleotide sequence encoding a heterologous invertase, wherein the invertase is bound to the plasma membrane of the thraustochytrid cell.

3. The cell according to claim 2, wherein the invertase is operably linked to a signal peptide.

4. The cell of any of claims 2-3, wherein the polynucleotide sequence encoding the heterologous invertase is at least 90% identical to the polynucleotide sequence of SEQ ID NO: 1.

5. The cell of any of claims 2-4, wherein the cell further comprises a nucleic acid molecule comprising a polynucleotide sequence encoding a heterologous sucrose transporter.

6. The cell of claim 5, wherein the polynucleotide sequence encoding the heterologous sucrose transporter is at least 90% identical to the polynucleotide sequence of Accession No. L47346.

7. The cell of any of claims 2-6, wherein the thraustochytrid is a *Schizochytrium* or a *Thraustochytrium.*

8. A thraustochytrid culture comprising:
(a) the thraustochytrid cell of any of clams 2-7, and
(b) a cell culture medium comprising sucrose as a carbon source.

9. A method of producing a thraustochytrid biomass, comprising:
(a) growing the thraustochytrid cell of any of claims 2-7 in a culture medium comprising sucrose as a carbon source, and
(b) harvesting the biomass from the culture medium.

10. A method of producing a microbial oil, comprising:
(a) growing the thraustochytrid cell of any of claims 2-7 in a culture medium comprising sucrose as a carbon source to produce a biomass, and
(b) extracting an oil from the biomass.

11. A method of producing a food product, cosmetic, industrial composition, or pharmaceutical composition for an animal or human, comprising:
(a) growing the thraustochytrid cell of any of claims 2-7 in a culture medium comprising sucrose as a carbon source,
(b) harvesting a biomass from the culture medium, and
(c) preparing the food product, cosmetic, industrial composition, or pharmaceutical composition from the biomass.

12. The method of claim 11, further comprising extracting an oil from the biomass and preparing the food product, cosmetic, industrial composition, or pharmaceutical composition from the oil.

13. The method of claim 11 or claim 12, wherein the food product is selected from (a) an infant formula, (b) milk, (c) a beverage, (d) a therapeutic drink, (e) a nutritional drink, (f) an additive for animal or human food, (g) a nutritional supplement, (h) an animal feed, and (i) a combination of (b), (c), (d), and/or (e).

## Patentansprüche

1. Verfahren zum Erzeugen einer Thraustochytriden-Zellkultur, das Folgendes umfasst:
(a) Transformieren einer Thraustochytridenzelle mit einem Nukleinsäuremolekül, das eine Polynukleotidsequenz, die für eine heterologe Invertase codiert, umfasst, wobei die Invertase an die Plasmamembran der Thraustochytridenzelle gebunden ist, und
(b) Züchten der transformierten Thraustochytridenzelle in einem Kulturmedium, das Saccharose als Kohlenstoffquelle umfasst.

2. Thraustochytridenzelle, umfassend ein Nukleinsäuremolekül, das eine Polynukleotidsequenz, die für eine heterologe Invertase codiert, umfasst, wobei die Invertase an die Plasmamembran der Thraustochytridenzelle gebunden ist.

3. Zelle nach Anspruch 2, wobei die Invertase operativ mit einem Signalpeptid verknüpft ist.

4. Zelle nach einem der Ansprüche 2-3, wobei die Polynukleotidsequenz, die für die heterologe Invertase codiert, zu mindestens 90% zu der Polynukleotidsequenz gemäß SEQ ID NO: 1 identisch ist.

5. Zelle nach einem der Ansprüche 2-4, wobei die Zelle weiterhin ein Nukleinsäuremolekül umfasst, das eine Polynukleotidsequenz, die für einen heterologen Saccharosetransporter codiert, umfasst.

6. Zelle nach Anspruch 5, wobei die Polynukleotidsequenz, die für den heterologen Saccharosetransporter codiert, zu mindestens 90% zu der Polynukleotidsequenz von Zugang Nr. L47346 identisch ist.

7. Zelle nach einem der Ansprüche 2-6, wobei es sich bei dem Thraustochytriden um ein *Schizochytrium* oder ein *Thraustochytrium* handelt.

8. Thraustochytridenkultur, die Folgendes umfasst:
(a) die Thraustochytridenzelle nach einem der Ansprüche 2-7 und
(b) ein Zellkulturmedium, das Saccharose als Kohlenstoffquelle umfasst.

9. Verfahren zum Produzieren einer Thraustochytridenbiomasse, das Folgendes umfasst:
(a) Züchten der Thraustochytridenzelle nach einem der Ansprüche 2-7 in einem Kulturmedium, das Saccarose als Kohlenstoffquelle umfasst und
(b) Ernten der Biomasse von dem Kulturmedium.

10. Verfahren zum Produzieren eines mikrobiellen Öls, das Folgendes umfasst:
(a) Züchten der Thraustochytridenzelle nach einem der Ansprüche 2-7 in einem Kulturmedium, das Saccarose als Kohlenstoffquelle umfasst, um eine Biomasse zu produzieren, und
(b) Extrahieren eines Öls aus der Biomasse.

11. Verfahren zum Erzeugen eines Nahrungsmittels, eines Kosmetikums, einer industriellen Zusammensetzung oder einer pharmazeutischen Zusammensetzung für ein Tier oder einen Menschen, das Folgendes umfasst:
(a) Züchten der Thraustochytridenzelle nach einem der Ansprüche 2-7 in einem Kulturmedium, das Saccharose als Kohlenstoffquelle umfasst,
(b) Ernten einer Biomasse von dem Kulturmedium und
(c) Erzeugen des Nahrungsmittels, des Kosmetikums, der industriellen Zusammensetzung oder der pharmazeutischen Zusammensetzung aus der Biomasse.

12. Verfahren nach Anspruch 11, das weiterhin Folgendes umfasst: Extrahieren eines Öls aus der Biomasse und Erzeugen des Nahrungsmittels, des Kosmetikums, der industriellen Zusammensetzung oder der pharmazeutischen Zusammensetzung aus dem Öl.

13. Verfahren nach Anspruch 11 oder Anspruch 12, wobei das Nahrungsmittel aus (a) einer Babynahrung, (b) Milch, (c) einem Getränk, (d) einem therapeutischen Drink, (e) einem Nähr-Drink, (f) einem Zusatzstoff für Tierfutter oder menschliche Nahrung, (g) einem Nahrungsergänzungsstoff, (h) einem Tierfutter und (i) einer Kombination von (b), (c), (d) und/oder (e) ausgewählt ist.

## Revendications

1. Procédé de production d'une culture de cellules de thraustochytride, comprenant :
(a) la transformation d'une cellule de thraustochytride avec une molécule d'acide nucléique comprenant une séquence polynucléotidique codant pour une invertase hétérologue, dans lequel l'invertase est liée à la membrane plasmique de la cellule de thraustochytride, et
(b) la croissance de la cellule de thraustochytride transformée dans un milieu de culture comprenant du saccharose en tant que source de carbone.

2. Cellule de thraustochytride comprenant une molécule d'acide nucléique comprenant une séquence polynucléotidique codant pour une invertase hétérologue, dans laquelle l'invertase est liée à la membrane plasmique de la cellule de thraustochytride.

3. Cellule selon la revendication 2, dans laquelle l'invertase est fonctionnellement liée à un peptide signal.

4. Cellule selon l'une quelconque des revendications 2 et 3, dans laquelle la séquence polynucléotidique codant pour l'invertase hétérologue est au moins 90 % identique à la séquence polynucléotidique de SEQ ID NO: 1.

5. Cellule selon l'une quelconque des revendications 2 à 4, la cellule comprenant en outre une molécule d'acide nucléique comprenant une séquence polynucléotidique codant pour un transporteur de saccharose hétérologue.

6. Cellule selon la revendication 5, dans laquelle la séquence polynucléotidique codant pour le transporteur de saccharose hétérologue est au moins 90 % identique à la séquence polynucléotidique d'accession n° L47346.

7. Cellule selon l'une quelconque des revendications 2 à 6, dans laquelle le thraustochytride est un *Schizochytrium* ou un *Thraustochytrium*.

8. Culture de thraustochytride comprenant :
(a) la cellule de thraustochytride selon l'une quelconque des revendications 2 à 7, et
(b) un milieu de culture de cellules comprenant du saccharose en tant que source de carbone.

9. Procédé de production d'une biomasse de thraustochytride, comprenant :
(a) la croissance de la cellule de thraustochytride selon l'une quelconque des revendications 2 à 7 dans un milieu de culture comprenant du saccharose en tant que source de carbone, et
(b) la collecte de la biomasse à partir du milieu de culture.

10. Procédé de production d'une huile microbienne, comprenant :
(a) la croissance de la cellule de thraustochytride selon l'une quelconque des revendications 2 à 7 dans un milieu de culture comprenant du saccharose en tant que source de carbone pour produire une biomasse, et
(b) l'extraction d'une huile à partir de la biomasse.

11. Procédé de production d'un produit alimentaire, d'un produit cosmétique, d'une composition industrielle ou d'une composition pharmaceutique pour un animal ou un humain, comprenant :
(a) la croissance de la cellule de thraustochytride selon l'une quelconque des revendications 2 à 7 dans un milieu de culture comprenant du saccharose en tant que source de carbone,
(b) la collecte d'une biomasse à partir du milieu de culture, et
(c) la préparation du produit alimentaire, du produit cosmétique, de la composition industrielle ou de la composition pharmaceutique à partir de la biomasse.

12. Procédé selon la revendication 11, comprenant en outre l'extraction d'une huile à partir de la biomasse et la préparation du produit alimentaire, du produit cosmétique, de la composition industrielle ou de la composition pharmaceutique à partir de l'huile.

13. Procédé selon la revendication 11 ou la revendication 12, dans lequel le produit alimentaire est choisi parmi (a) une formule pour nourrissons, (b) du lait, (c) une boisson, (d) une boisson thérapeutique, (e) une boisson nutritionnelle, (f) un additif pour alimentation animale ou humaine, (g) un complément nutritionnel, (h) un aliment pour animaux, et (i) une combinaison de (b), (c), (d) et/ou e).


### SEQUENCE LISTING

<110> MARTEK BIOSCIENCES CORPORATION
<120> Recombinant Thraustochytrids That Grow on Sucrose, and Compositions, Methods of Making, and Uses thereof
<130> 2715.046PC01/JUK/SAS/BNC
<140> To be assigned
   <141> Herewith
<150> 61/290,443
   <151> 2009-12-28
<160> 10
<170> PatentIn version 3.3
<210> 1
   <211> 1614
   <212> DNA
   <213> Artificial sequence
<220>
   <223> pSCHIZ Secl-Invertase
<400> 1
   ggatccatga agttcgcgac ctcggtcgca attttgcttg tggccaacat agccaccgcc      60
   ctcgcgtcga tgaccaacga gacctcggac cgccctctcg tgcactttac ccccaacaag     120
   ggttggatga acgatcccaa cggcctctgg tacgacgaga aggatgctaa gtggcacctt     180
   tactttcagt acaaccctaa cgacaccgtc tggggcaccc cgctcttctg gggccacgcc     240
   acctccgacg acctcaccaa ctgggaggac cagcccattg ctatcgcccc caagcgcaac     300
   gactcgggag ctttttccgg ttccatggtt gtggactaca acaacacctc cggttttttt     360
   aacgacacca ttgacccccg ccagcgctgc gtcgccatct ggacctacaa cacgcccgag     420
   agcgaggagc agtacatcag ctacagcctt gatggaggct acacctttac cgagtaccag     480
   aagaaccctg tcctcgccgc caactccacc cagttccgcg accctaaggt tttttggtac     540
   gagccttccc agaagtggat tatgaccgcc gctaagtcgc aggattacaa gatcgagatc     600
   tacagcagcg acgacctcaa gtcctggaag cttgagtccg cctttgccaa cgagggtttt     660
   ctcggatacc agtacgagtg ccccggtctc atcgaggtcc ccaccgagca ggacccgtcc     720
   aagtcctact gggtcatgtt tatttccatc aaccctggcg cccctgccgg cggcagcttc     780
   aaccagtact tcgtcggctc ctttaacggc acgcattttg aggccttcga caaccagtcc     840
   cgcgtcgtcg acttcggcaa ggactactac gccctccaga ccttctttaa caccgacccc     900
   acctacggca gcgccctcgg tattgcttgg gcctccaact gggagtactc cgctttcgtc     960
   cccactaacc cctggcgcag ctcgatgtcc ctcgtccgca agttttcgct taacaccgag    1020
   taccaggcca accccgagac cgagcttatt aacctgaagg ccgagcctat tctcaacatc    1080
   tccaacgctg gcccctggtc ccgctttgct actaacacta ccctcaccaa ggccaactcc    1140
   tacaacgtcg atctctccaa ctccaccggt actcttgagt ttgagctcgt ctacgccgtc    1200
   aacaccaccc agaccatctc caagtccgtc ttcgccgacc tctccctctg gttcaagggc    1260
   cttgaggacc ccgaggagta cctgcgcatg ggttttgagg tctccgcctc ctccttcttc    1320
   ctcgatcgcg gtaactccaa ggttaagttt gtcaaggaga acccctactt tactaaccgt    1380
   atgagcgtca acaaccagcc ctttaagtcc gagaacgatc ttagctacta caaggtttac    1440
   ggcctcctcg accagaacat tctcgagctc tactttaacg acggagatgt cgtcagcacc    1500
   aacacctact ttatgaccac tggaaacgcc ctcggcagcg tgaacatgac caccggagtc    1560
   gacaacctct tttacattga caagtttcag gttcgcgagg ttaagtaaca tatg          1614
<210> 2
   <211> 11495
   <212> DNA
   <213> Artificial sequence
<220>
   <223> pCL0076
<400> 2
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg gggggagggg agaggcagga     840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata     960
   gtatcttgcg tgcttggacg aggagactga cgaggaggac ggatacgtcg atgatgatgt    1020
   gcacagagaa gaagcagttc gaaagcgact actagcaagc aagggatcca tgaagttcgc 1080
   gacctcggtc gcaattttgc ttgtggccaa catagccacc gccctcgcgt cgatgaccaa 1140
   cgagacctcg gaccgccctc tcgtgcactt tacccccaac aagggttgga tgaacgatcc 1200
   caacggcctc tggtacgacg agaaggatgc taagtggcac ctttactttc agtacaaccc 1260
   taacgacacc gtctggggca ccccgctctt ctggggccac gccacctccg acgacctcac 1320
   caactgggag gaccagccca ttgctatcgc ccccaagcgc aacgactcgg gagctttttc 1380
   cggttccatg gttgtggact acaacaacac ctccggtttt tttaacgaca ccattgaccc 1440
   ccgccagcgc tgcgtcgcca tctggaccta caacacgccc gagagcgagg agcagtacat 1500
   cagctacagc cttgatggag gctacacctt taccgagtac cagaagaacc ctgtcctcgc 1560
   cgccaactcc acccagttcc gcgaccctaa ggttttttgg tacgagcctt cccagaagtg 1620
   gattatgacc gccgctaagt cgcaggatta caagatcgag atctacagca gcgacgacct 1680
   caagtcctgg aagcttgagt ccgcctttgc caacgagggt tttctcggat accagtacga 1740
   gtgccccggt ctcatcgagg tccccaccga gcaggacccg tccaagtcct actgggtcat 1800
   gtttatttcc atcaaccctg gcgcccctgc cggcggcagc ttcaaccagt acttcgtcgg 1860
   ctcctttaac ggcacgcatt ttgaggcctt cgacaaccag tcccgcgtcg tcgacttcgg 1920
   caaggactac tacgccctcc agaccttctt taacaccgac cccacctacg gcagcgccct 1980
   cggtattgct tgggcctcca actgggagta ctccgctttc gtccccacta acccctggcg 2040
   cagctcgatg tccctcgtcc gcaagttttc gcttaacacc gagtaccagg ccaaccccga 2100
   gaccgagctt attaacctga aggccgagcc tattctcaac atctccaacg ctggcccctg 2160
   gtcccgcttt gctactaaca ctaccctcac caaggccaac tcctacaacg tcgatctctc 2220
   caactccacc ggtactcttg agtttgagct cgtctacgcc gtcaacacca cccagaccat 2280
   ctccaagtcc gtcttcgccg acctctccct ctggttcaag ggccttgagg accccgagga 2340
   gtacctgcgc atgggttttg aggtctccgc ctcctccttc ttcctcgatc gcggtaactc 2400
   caaggttaag tttgtcaagg agaaccccta ctttactaac cgtatgagcg tcaacaacca 2460
   gccctttaag tccgagaacg atcttagcta ctacaaggtt tacggcctcc tcgaccagaa 2520
   cattctcgag ctctacttta acgacggaga tgtcgtcagc accaacacct actttatgac 2580
   cactggaaac gccctcggca gcgtgaacat gaccaccgga gtcgacaacc tcttttacat 2640
   tgacaagttt caggttcgcg aggttaagta acatatgtta tgagagatcc gaaagtgaac 2700
   cttgtcctaa cccgacagcg aatggcggga gggggcgggc taaaagatcg tattacatag 2760
   tatttttccc ctactctttg tgtttgtctt tttttttttt ttgaacgcat tcaagccact 2820
   tgtctgggtt tacttgtttg tttgcttgct tgcttgcttg cttgcctgct tcttggtcag 2880
   acggcccaaa aaagggaaaa aattcattca tggcacagat aagaaaaaga aaaagtttgt 2940
   cgaccaccgt catcagaaag caagagaaga gaaacactcg cgctcacatt ctcgctcgcg 3000
   taagaatctt agccacgcat acgaagtaat ttgtccatct ggcgaatctt tacatgagcg 3060
   ttttcaagct ggagcgtgag atcatacctt tcttgatcgt aatgttccaa ccttgcatag 3120
   gcctcgttgc gatccgctag caatgcgtcg tactcccgtt gcaactgcgc catcgcctca 3180
   ttgtgacgtg agttcagatt cttctcgaga ccttcgagcg ctgctaattt cgcctgacgc 3240
   tccttctttt gtgcttccat gacacgccgc ttcaccgtgc gttccacttc ttcctcagac 3300
   atgcccttgg ctgcctcgac ctgctcggta agcttcgtcg taatctcctc gatctcggaa 3360
   ttcttcttgc cctccatcca ctcggcacca tacttggcag cctgttcaac acgctcattg 3420
   aaaaactttt cattctcttc cagctccgca acccgcgctc gaagctcatt cacttccgcc 3480
   accacggctt cggcatcgag cgccgaatca gtcgccgaac tttccgaaag atacaccacg 3540
   gcccctccgc tgctgctgcg cagcgtcatc atcagtcgcg tgttatcttc gcgcagattc 3600
   tccacctgct ccgtaagcag cttcacggtg gcctcttgat tctgagggct cacgtcgtgg 3660
   attagcgctt gcagctcttg cagctccgtc agcttggaag agctcgtaat catggctttg 3720
   cacttgtcca gacgtcgcag agcgttcgag agccgcttcg cgttatctgc catggacgct 3780
   tctgcgctcg cggcctccct gacgacagtc tcttgcagtt tcactagatc atgtccaatc 3840
   agcttgcggt gcagctctcc aatcacgttc tgcatcttgt ttgtgtgtcc gggccgcgcc 3900
   tcgtcttgcg atttgcgaat ttcctcctcg agctcgcgtt cgagctccag ggcgccttta 3960
   agtagctcga agtcagccgc cgttagcccc agctccgtcg ccgcgttcag acagtcggtt 4020
   agcttgattc gattccgctt ttccatggca agtttaagat cctggcccag ctgcacctcc 4080
   tgcgccttgc gcatcatgcg cggttccgcc tggcgcaaaa gcttcgagtc gtatcctgcc 4140
   tgccatgcca gcgcaatggc acgcacgagc gacttgagtt gccaactatt catcgccgag 4200
   atgagcagca ttttgatctg catgaacacc tcgtcagagt cgtcatcctc tgcctcctcc 4260
   agctctgcgg gcgagcgacg ctctccttgc agatgaagcg agggccgcag gcctccgaag 4320
   agcacctctt gcgcgagatc ctcctccgtc gtcgccctcc gcaggattgc ggtcgtgtcc 4380
   gccatcttgc cgccacagca gcttttgctc gctctgcacc ttcaatttct ggtgccgctg 4440
   gtgccgctgg tgccgcttgt gctggtgctg gtgctggtgc tggtgctggt gccttgtgct 4500
   ggtgctgcca cagacaccgc cgctcctgct gctgctcttc cggccccctc gccgccgccg 4560
   cgagcccccg ccgcgcgccg tgcctgggct ctccgcgctc tccgcgggct cctcggcctc 4620
   ggcctcgccg tccgcgacga cgtctgcgcg gccgatggtg cggatctgct ctagagggcc 4680
   cttcgaaggt aagcctatcc ctaaccctct cctcggtctc gattctacgc gtaccggtca 4740
   tcatcaccat caccattgag tttaaacggg ccccagcacg tgctacgaga tttcgattcc 4800
   accgccgcct tctatgaaag gttgggcttc ggaatcgttt tccgggacgc cggctggatg 4860
   atcctccagc gcggggatct catgctggag ttcttcgccc accccaactt gtttattgca 4920
   gcttataatg gttacaaata aagcaatagc atcacaaatt tcacaaataa agcatttttt 4980
   tcactgcatt ctagttgtgg tttgtccaaa ctcatcaatg tatcttatca tacatggtcg 5040
   acctgcagga acctgcatta atgaatcggc caacgcgcgg ggagaggcgg tttgcgtatt 5100
   gggcgctctt ccgcttcctc gctcactgac tcgctgcgct cggtcgttcg gctgcggcga 5160
   gcggtatcag ctcactcaaa ggcggtaata cggttatcca cagaatcagg ggataacgca 5220
   ggaaagaaca tgtgagcaaa aggccagcaa aaggccagga accgtaaaaa ggccgcgttg 5280
   ctggcgtttt tccataggct ccgcccccct gacgagcatc acaaaaatcg acgctcaagt 5340
   cagaggtggc gaaacccgac aggactataa agataccagg cgtttccccc tggaagctcc 5400
   ctcgtgcgct ctcctgttcc gaccctgccg cttaccggat acctgtccgc ctttctccct 5460
   tcgggaagcg tggcgctttc tcatagctca cgctgtaggt atctcagttc ggtgtaggtc 5520
   gttcgctcca agctgggctg tgtgcacgaa ccccccgttc agcccgaccg ctgcgcctta 5580
   tccggtaact atcgtcttga gtccaacccg gtaagacacg acttatcgcc actggcagca 5640
   gccactggta acaggattag cagagcgagg tatgtaggcg gtgctacaga gttcttgaag 5700
   tggtggccta actacggcta cactagaaga acagtatttg gtatctgcgc tctgctgaag 5760
   ccagttacct tcggaaaaag agttggtagc tcttgatccg gcaaacaaac caccgctggt 5820
   agcggtggtt tttttgtttg caagcagcag attacgcgca gaaaaaaagg atctcaagaa 5880
   gatcctttga tcttttctac ggggtctgac gctcagtgga acgaaaactc acgttaaggg 5940
   attttggtca tgagattatc aaaaaggatc ttcacctaga tccttttaaa ttaaaaatga 6000
   agttttaaat caatctaaag tatatatgag taaacttggt ctgacagtta ccaatgctta 6060
   atcagtgagg cacctatctc agcgatctgt ctatttcgtt catccatagt tgcctgactc 6120
   cccgtcgtgt agataactac gatacgggag ggcttaccat ctggccccag tgctgcaatg 6180
   ataccgcgag acccacgctc accggctcca gatttatcag caataaacca gccagccgga 6240
   agggccgagc gcagaagtgg tcctgcaact ttatccgcct ccatccagtc tattaattgt 6300
   tgccgggaag ctagagtaag tagttcgcca gttaatagtt tgcgcaacgt tgttgccatt 6360
   gctacaggca tcgtggtgtc acgctcgtcg tttggtatgg cttcattcag ctccggttcc 6420
   caacgatcaa ggcgagttac atgatccccc atgttgtgca aaaaagcggt tagctccttc 6480
   ggtcctccga tcgttgtcag aagtaagttg gccgcagtgt tatcactcat ggttatggca 6540
   gcactgcata attctcttac tgtcatgcca tccgtaagat gcttttctgt gactggtgag 6600
   tactcaacca agtcattctg agaatagtgt atgcggcgac cgagttgctc ttgcccggcg 6660
   tcaatacggg ataataccgc gccacatagc agaactttaa aagtgctcat cattggaaaa 6720
   cgttcttcgg ggcgaaaact ctcaaggatc ttaccgctgt tgagatccag ttcgatgtaa 6780
   cccactcgtg cacccaactg atcttcagca tcttttactt tcaccagcgt ttctgggtga 6840
   gcaaaaacag gaaggcaaaa tgccgcaaaa aagggaataa gggcgacacg gaaatgttga 6900
   atactcatac tcttcctttt tcaatattat tgaagcattt atcagggtta ttgtctcatg 6960
   agcggataca tatttgaatg tatttagaaa aataaacaaa taggggttcc gcgcacattt 7020
   ccccgaaaag tgccacctga cgtctaagaa accattatta tcatgacatt aacctataaa 7080
   aataggcgta tcacgaggcc ctttcgtctc gcgcgtttcg gtgatgacgg tgaaaacctc 7140
   tgacacatgc agctcccgga gacggtcaca gcttgtctgt aagcggatgc cgggagcaga 7200
   caagcccgtc agggcgcgtc agcgggtgtt ggcgggtgtc ggggctggct taactatgcg 7260
   gcatcagagc agattgtact gagagtgcac caagctttgc ctcaacgcaa ctaggcccag 7320
   gcctactttc actgtgtctt gtcttgcctt tcacaccgac cgagtgtgca caaccgtgtt 7380
   ttgcacaaag cgcaagatgc tcactcgact gtgaagcaaa ggttgcgcgc aagcgactgc 7440
   gactgcgagg atgaggatga ctggcagcct gttcaaaaac tgaaaatccg cgatgggtca 7500
   gctgccattc gcgcatgacg cctgcgagag acaagttaac tcgtgtcact ggcatgtcct 7560
   agcatcttta cgcgagcaaa attcaatcgc tttatttttt cagtttcgta accttctcgc 7620
   aaccgcgaat cgccgtttca gcctgactaa tctgcagctg cgtggcactg tcagtcagtc 7680
   agtcagtcgt gcgcgctgtt ccagcaccga ggtcgcgcgt cgccgcgcct ggaccgctgc 7740
   tgctactgct agtggcacgg caggtaggag cttgttgccg gaacaccagc agccgccagt 7800
   cgacgccagc caggggaaag tccggcgtcg aagggagagg aaggcggcgt gtgcaaacta 7860
   acgttgacca ctggcgcccg ccgacacgag caggaagcag gcagctgcag agcgcagcgc 7920
   gcaagtgcag aatgcgcgaa agatccactt gcgcgcggcg ggcgcgcact tgcgggcgcg 7980
   gcgcggaaca gtgcggaaag gagcggtgca gacggcgcgc agtgacagtg ggcgcaaagc 8040
   cgcgcagtaa gcagcggcgg ggaacggtat acgcagtgcc gcgggccgcc gcacacagaa 8100
   gtatacgcgg gccgaagtgg ggcgtcgcgc gcgggaagtg cggaatggcg ggcaaggaaa 8160
   ggaggagacg gaaagagggc gggaaagaga gagagagaga gtgaaaaaag aaagaaagaa 8220
   agaaagaaag aaagaaagct cggagccacg ccgcggggag agagagaaat gaaagcacgg 8280
   cacggcaaag caaagcaaag cagacccagc cagacccagc cgagggagga gcgcgcgcag 8340
   gacccgcgcg gcgagcgagc gagcacggcg cgcgagcgag cgagcgagcg agcgcgcgag 8400
   cgagcaaggc ttgctgcgag cgatcgagcg agcgagcggg aaggatgagc gcgacccgcg 8460
   cggcgacgag gacagcggcg gcgctgtcct cggcgctgac gacgcctgta aagcagcagc 8520
   agcagcagca gctgcgcgta ggcgcggcgt cggcacggct ggcggccgcg gcgttctcgt 8580
   ccggcacggg cggagacgcg gccaagaagg cggccgcggc gagggcgttc tccacgggac 8640
   gcggccccaa cgcgacacgc gagaagagct cgctggccac ggtccaggcg gcgacggacg 8700
   atgcgcgctt cgtcggcctg accggcgccc aaatctttca tgagctcatg cgcgagcacc 8760
   aggtggacac catctttggc taccctggcg gcgccattct gcccgttttt gatgccattt 8820
   ttgagagtga cgcgcttcaa gttcattctc gctcgccacg agcagggcgc cggccacatg 8880
   gccgagggct acgcgcgcgc cacgggcaag cccggcgttg tcctcgtcac ctcgggccct 8940
   ggagccacca acaccatcac cccgatcatg gatgcttaca tggacggtac gccgctgctc 9000
   gtgttcaccg gccaggtgca gacctctgct gtcggcacgg acgctttcca ggagtgtgac 9060
   attgttggca tcagccgcgc gtgcaccaag tggaacgtca tggtcaagga cgtgaaggag 9120
   ctcccgcgcc gcatcaatga ggcctttgag attgccatga gcggccgccc gggtcccgtg 9180
   ctcgtcgatc ttcctaagga tgtgaccgcc gttgagctca aggaaatgcc cgacagctcc 9240
   ccccaggttg ctgtgcgcca gaagcaaaag gtcgagcttt tccacaagga gcgcattggc 9300
   gctcctggca cggccgactt caagctcatt gccgagatga tcaaccgtgc ggagcgaccc 9360
   gtcatctatg ctggccaggg tgtcatgcag agcccgttga atggcccggc tgtgctcaag 9420
   gagttcgcgg agaaggccaa cattcccgtg accaccacca tgcagggtct cggcggcttt 9480
   gacgagcgta gtcccctctc cctcaagatg ctcggcatgc acggctctgc ctacgccaac 9540
   tactcgatgc agaacgccga tcttatcctg gcgctcggtg cccgctttga tgatcgtgtg 9600
   acgggccgcg ttgacgcctt tgctccggag gctcgccgtg ccgagcgcga gggccgcggt 9660
   ggcatcgttc actttgagat ttcccccaag aacctccaca aggtcgtcca gcccaccgtc 9720
   gcggtcctcg gcgacgtggt cgagaacctc gccaacgtca cgccccacgt gcagcgccag 9780
   gagcgcgagc cgtggtttgc gcagatcgcc gattggaagg agaagcaccc ttttctgctc 9840
   gagtctgttg attcggacga caaggttctc aagccgcagc aggtcctcac ggagcttaac 9900
   aagcagattc tcgagattca ggagaaggac gccgaccagg aggtctacat caccacgggc 9960
   gtcggaagcc accagatgca ggcagcgcag ttccttacct ggaccaagcc gcgccagtgg 10020
   atctcctcgg gtggcgccgg cactatgggc tacggccttc cctcggccat tggcgccaag 10080
   attgccaagc ccgatgctat tgttattgac atcgatggtg atgcttctta ttcgatgacc 10140
   ggtatggaat tgatcacagc agccgaattc aaggttggcg tgaagattct tcttttgcag 10200
   aacaactttc agggcatggt caagaacgtt caggatctct tttacgacaa gcgctactcg 10260
   ggccaccgcc atgttcaacc cgcgcttcga caaggtcgcc gatgcgatgc gtgccaaggg 10320
   tctctactgc gcgaaacagt cggagctcaa ggacaagatc aaggagtttc tcgagtacga 10380
   tgagggtccc gtcctcctcg aggttttcgt ggacaaggac acgctcgtct tgcccatggt 10440
   ccccgctggc tttccgctcc acgagatggt cctcgagcct cctaagccca aggacgccta 10500
   agttcttttt tccatggcgg gcgagcgagc gagcgcgcga gcgcgcaagt gcgcaagcgc 10560
   cttgccttgc tttgcttcgc ttcgctttgc tttgcttcac acaacctaag tatgaattca 10620
   agttttcttg cttgtcggcg atgcctgcct gccaaccagc cagccatccg gccggccgtc 10680
   cttgacgcct tcgcttccgg cgcggccatc gattcaattc acccatccga tacgttccgc 10740
   cccctcacgt ccgtctgcgc acgacccctg cacgaccacg ccaaggccaa cgcgccgctc 10800
   agctcagctt gtcgacgagt cgcacgtcac atatctcaga tgcatttgga ctgtgagtgt 10860
   tattatgcca ctagcacgca acgatcttcg gggtcctcgc tcattgcatc cgttcgggcc 10920
   ctgcaggcgt ggacgcgagt cgccgccgag acgctgcagc aggccgctcc gacgcgaggg 10980
   ctcgagctcg ccgcgcccgc gcgatgtctg cctggcgccg actgatctct ggagcgcaag 11040
   gaagacacgg cgacgcgagg aggaccgaag agagacgctg gggtatgcag gatatacccg 11100
   gggcgggaca ttcgttccgc atacactccc ccattcgagc ttgctcgtcc ttggcagagc 11160
   cgagcgcgaa cggttccgaa cgcggcaagg attttggctc tggtgggtgg actccgatcg 11220
   aggcgcaggt tctccgcagg ttctcgcagg ccggcagtgg tcgttagaaa tagggagtgc 11280
   cggagtcttg acgcgcctta gctcactctc cgcccacgcg cgcatcgccg ccatgccgcc 11340
   gtcccgtctg tcgctgcgct ggccgcgacc ggctgcgcca gagtacgaca gtgggacaga 11400
   gctcgaggcg acgcgaatcg ctcgggttgt aagggtttca agggtcgggc gtcgtcgcgt 11460
   gccaaagtga aaatagtagg gggggggggg ggtac 11495
<210> 3
   <211> 20
   <212> PRT
   <213> Schizochytrium
<400> 3
   Met Lys Phe Ala Thr Ser Val Ala Ile Leu Leu Val Ala Asn Ile Ala
   1               5                   10                  15
   Thr Ala Leu Ala
               20
<210> 4
   <211> 1785
   <212> DNA
   <213> Artificial
<220>
   <223> Codon optimized nucleic acid sequence
<400> 4
   atgaagttcg cgacctcggt cgcaattttg cttgtggcca acatagccac cgccctcgcg 60
   gcctccccct cgatgcagac ccgtgcctcc gtcgtcattg attacaacgt cgctcctcct 120
   aacctctcca ccctcccgaa cggcagcctc tttgagacct ggcgtcctcg cgcccacgtt 180
   cttcccccta acggtcagat tggcgatccc tgcctccact acaccgatcc ctcgactggc 240
   ctctttcacg tcggctttct ccacgatggc tccggcattt cctccgccac tactgacgac 300
   ctcgctacct acaaggatct caaccagggc aaccaggtca tcgtccccgg cggtatcaac 360
   gaccctgtcg ctgttttcga cggctccgtc attccttccg gcattaacgg cctccctacc 420
   ctcctctaca cctccgtcag ctacctcccc attcactggt ccatccccta cacccgcggt 480
   tccgagacgc agagcctggc tgtctccagc gatggtggct ccaactttac taagctcgac 540
   cagggccccg ttattcctgg cccccccttt gcctacaacg tcaccgcctt ccgcgacccc 600
   tacgtctttc agaaccccac cctcgactcc ctcctccact ccaagaacaa cacctggtac 660
   accgtcattt cgggtggcct ccacggcaag ggccccgccc agtttcttta ccgtcagtac 720
   gaccccgact ttcagtactg ggagttcctc ggccagtggt ggcacgagcc taccaactcc 780
   acctggggca acggcacctg ggccggccgc tgggccttca acttcgagac cggcaacgtc 840
   ttttcgcttg acgagtacgg ctacaacccc cacggccaga tcttctccac cattggcacc 900
   gagggctccg accagcccgt tgtcccccag ctcacctcca tccacgatat gctttgggtc 960
   tccggtaacg tttcgcgcaa cggatcggtt tccttcactc ccaacatggc cggcttcctc 1020
   gactggggtt tctcgtccta cgccgccgcg ggtaaggttc ttccttccac gtcgctcccc 1080
   tccaccaagt ccggtgcccc cgatcgcttc atttcgtacg tttggctctc cggcgacctc 1140
   tttgagcagg ctgagggctt tcctaccaac cagcagaact ggaccggcac cctcctcctc 1200
   ccccgtgagc tccgcgtcct ttacatcccc aacgtggttg ataacgccct tgcgcgcgag 1260
   tccggcgctt cctggcaggt cgtctcctcc gatagctcgg ccggtactgt ggagctccag 1320
   accctcggca tttccatcgc ccgcgagacc aaggccgccc tcctgtccgg cacctcgttc 1380
   actgagtccg accgcactct taactcctcc ggcgtcgttc cctttaagcg ttccccctcc 1440
   gagaagtttt tcgtcctctc cgcccagctc tccttccccg cctccgcccg cggctcgggc 1500
   ctcaagtccg gcttccagat tctttcctcc gagctcgagt ccaccacggt ctactaccag 1560
   tttagcaacg agtccatcat cgtcgaccgc agcaacacca gcgccgccgc ccgtactacc 1620
   gacggtatcg actcctccgc cgaggccggc aagctccgcc tctttgacgt cctcaacggc 1680
   ggcgagcagg ctattgagac cctcgacctt accctcgtcg ttgataactc cgtgctcgag 1740
   atttacgcca acggtcgttt cgcgctttcc acctgggttc gctaa 1785
<210> 5
   <211> 9291
   <212> DNA
   <213> Artificial Sequence
<220>
   <223> pCL0120
<400> 5
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc 120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg gggggggggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaagttcgc 1080
   gacctcggtc gcaattttgc ttgtggccaa catagccacc gccctcgcgc agagcgatgg 1140
   ctgcaccccc accgaccaga cgatggtgag caagggcgag gagctgttca ccggggtggt 1200
   gcccatcctg gtcgagctgg acggcgacgt aaacggccac aagttcagcg tgtccggcga 1260
   gggcgagggc gatgccacct acggcaagct gaccctgaag ttcatctgca ccaccggcaa 1320
   gctgcccgtg ccctggccca ccctcgtgac caccctgacc tacggcgtgc agtgcttcag 1380
   ccgctacccc gaccacatga agcagcacga cttcttcaag tccgccatgc ccgaaggcta 1440
   cgtccaggag cgcaccatct tcttcaagga cgacggcaac tacaagaccc gcgccgaggt 1500
   gaagttcgag ggcgacaccc tggtgaaccg catcgagctg aagggcatcg acttcaagga 1560
   ggacggcaac atcctgggac acaagctgga gtacaactac aacagccaca acgtctatat 1620
   catggccgac aagcagaaga acggcatcaa ggtgaacttc aagatccgcc acaacatcga 1680
   ggacggcagc gtgcagctcg ccgaccacta ccagcagaac acccccatcg gcgacggccc 1740
   cgtgctgctg cccgacaacc actacctgag cacccagtcc gccctgagca aagaccccaa 1800
   cgagaagcgc gatcacatgg tcctgctgga gttcgtgacc gccgccggga tcactctcgg 1860
   catggacgag ctgtacaagc accaccatca ccaccactaa catatgagtt atgagatccg 1920
   aaagtgaacc ttgtcctaac ccgacagcga atggcgggag ggggcgggct aaaagatcgt 1980
   attacatagt atttttcccc tactctttgt gtttgtcttt tttttttttt tgaacgcatt 2040
   caagccactt gtctgggttt acttgtttgt ttgcttgctt gcttgcttgc ttgcctgctt 2100
   cttggtcaga cggcccaaaa aagggaaaaa attcattcat ggcacagata agaaaaagaa 2160
   aaagtttgtc gaccaccgtc atcagaaagc aagagaagag aaacactcgc gctcacattc 2220
   tcgctcgcgt aagaatctta gccacgcata cgaagtaatt tgtccatctg gcgaatcttt 2280
   acatgagcgt tttcaagctg gagcgtgaga tcataccttt cttgatcgta atgttccaac 2340
   cttgcatagg cctcgttgcg atccgctagc aatgcgtcgt actcccgttg caactgcgcc 2400
   atcgcctcat tgtgacgtga gttcagattc ttctcgagac cttcgagcgc tgctaatttc 2460
   gcctgacgct ccttcttttg tgcttccatg acacgccgct tcaccgtgcg ttccacttct 2520
   tcctcagaca tgcccttggc tgcctcgacc tgctcggtaa aacgggcccc agcacgtgct 2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag ctttgcctca 5100
   acgcaactag gcccaggcct actttcactg tgtcttgtct tgcctttcac accgaccgag 5160
   tgtgcacaac cgtgttttgc acaaagcgca agatgctcac tcgactgtga agcaaaggtt 5220
   gcgcgcaagc gactgcgact gcgaggatga ggatgactgg cagcctgttc aaaaactgaa 5280
   aatccgcgat gggtcagctg ccattcgcgc atgacgcctg cgagagacaa gttaactcgt 5340
   gtcactggca tgtcctagca tctttacgcg agcaaaattc aatcgcttta ttttttcagt 5400
   ttcgtaacct tctcgcaacc gcgaatcgcc gtttcagcct gactaatctg cagctgcgtg 5460
   gcactgtcag tcagtcagtc agtcgtgcgc gctgttccag caccgaggtc gcgcgtcgcc 5520
   gcgcctggac cgctgctgct actgctagtg gcacggcagg taggagcttg ttgccggaac 5580
   accagcagcc gccagtcgac gccagccagg ggaaagtccg gcgtcgaagg gagaggaagg 5640
   cggcgtgtgc aaactaacgt tgaccactgg cgcccgccga cacgagcagg aagcaggcag 5700
   ctgcagagcg cagcgcgcaa gtgcagaatg cgcgaaagat ccacttgcgc gcggcgggcg 5760
   cgcacttgcg ggcgcggcgc ggaacagtgc ggaaaggagc ggtgcagacg gcgcgcagtg 5820
   acagtgggcg caaagccgcg cagtaagcag cggcggggaa cggtatacgc agtgccgcgg 5880
   gccgccgcac acagaagtat acgcgggccg aagtggggcg tcgcgcgcgg gaagtgcgga 5940
   atggcgggca aggaaaggag gagacggaaa gagggcggga aagagagaga gagagagtga 6000
   aaaaagaaag aaagaaagaa agaaagaaag aaagctcgga gccacgccgc ggggagagag 6060
   agaaatgaaa gcacggcacg gcaaagcaaa gcaaagcaga cccagccaga cccagccgag 6120
   ggaggagcgc gcgcaggacc cgcgcggcga gcgagcgagc acggcgcgcg agcgagcgag 6180
   cgagcgagcg cgcgagcgag caaggcttgc tgcgagcgat cgagcgagcg agcgggaagg 6240
   atgagcgcga cccgcgcggc gacgaggaca gcggcggcgc tgtcctcggc gctgacgacg 6300
   cctgtaaagc agcagcagca gcagcagctg cgcgtaggcg cggcgtcggc acggctggcg 6360
   gccgcggcgt tctcgtccgg cacgggcgga gacgcggcca agaaggcggc cgcggcgagg 6420
   gcgttctcca cgggacgcgg ccccaacgcg acacgcgaga agagctcgct ggccacggtc 6480
   caggcggcga cggacgatgc gcgcttcgtc ggcctgaccg gcgcccaaat ctttcatgag 6540
   ctcatgcgcg agcaccaggt ggacaccatc tttggctacc ctggcggcgc cattctgccc 6600
   gtttttgatg ccatttttga gagtgacgcg cttcaagttc attctcgctc gccacgagca 6660
   gggcgccggc cacatggccg agggctacgc gcgcgccacg ggcaagcccg gcgttgtcct 6720
   cgtcacctcg ggccctggag ccaccaacac catcaccccg atcatggatg cttacatgga 6780
   cggtacgccg ctgctcgtgt tcaccggcca ggtgcagacc tctgctgtcg gcacggacgc 6840
   tttccaggag tgtgacattg ttggcatcag ccgcgcgtgc accaagtgga acgtcatggt 6900
   caaggacgtg aaggagctcc cgcgccgcat caatgaggcc tttgagattg ccatgagcgg 6960
   ccgcccgggt cccgtgctcg tcgatcttcc taaggatgtg accgccgttg agctcaagga 7020
   aatgcccgac agctcccccc aggttgctgt gcgccagaag caaaaggtcg agcttttcca 7080
   caaggagcgc attggcgctc ctggcacggc cgacttcaag ctcattgccg agatgatcaa 7140
   ccgtgcggag cgacccgtca tctatgctgg ccagggtgtc atgcagagcc cgttgaatgg 7200
   cccggctgtg ctcaaggagt tcgcggagaa ggccaacatt cccgtgacca ccaccatgca 7260
   gggtctcggc ggctttgacg agcgtagtcc cctctccctc aagatgctcg gcatgcacgg 7320
   ctctgcctac gccaactact cgatgcagaa cgccgatctt atcctggcgc tcggtgcccg 7380
   ctttgatgat cgtgtgacgg gccgcgttga cgcctttgct ccggaggctc gccgtgccga 7440
   gcgcgagggc cgcggtggca tcgttcactt tgagatttcc cccaagaacc tccacaaggt    7500
   cgtccagccc accgtcgcgg tcctcggcga cgtggtcgag aacctcgcca acgtcacgcc    7560
   ccacgtgcag cgccaggagc gcgagccgtg gtttgcgcag atcgccgatt ggaaggagaa    7620
   gcaccctttt ctgctcgagt ctgttgattc ggacgacaag gttctcaagc cgcagcaggt    7680
   cctcacggag cttaacaagc agattctcga gattcaggag aaggacgccg accaggaggt    7740
   ctacatcacc acgggcgtcg gaagccacca gatgcaggca gcgcagttcc ttacctggac    7800
   caagccgcgc cagtggatct cctcgggtgg cgccggcact atgggctacg gccttccctc    7860
   ggccattggc gccaagattg ccaagcccga tgctattgtt attgacatcg atggtgatgc    7920
   ttcttattcg atgaccggta tggaattgat cacagcagcc gaattcaagg ttggcgtgaa    7980
   gattcttctt ttgcagaaca actttcaggg catggtcaag aacgttcagg atctctttta    8040
   cgacaagcgc tactcgggcc accgccatgt tcaacccgcg cttcgacaag gtcgccgatg    8100
   cgatgcgtgc caagggtctc tactgcgcga aacagtcgga gctcaaggac aagatcaagg    8160
   agtttctcga gtacgatgag ggtcccgtcc tcctcgaggt tttcgtggac aaggacacgc    8220
   tcgtcttgcc catggtcccc gctggctttc cgctccacga gatggtcctc gagcctccta    8280
   agcccaagga cgcctaagtt cttttttcca tggcgggcga gcgagcgagc gcgcgagcgc    8340
   gcaagtgcgc aagcgccttg ccttgctttg cttcgcttcg ctttgctttg cttcacacaa    8400
   cctaagtatg aattcaagtt ttcttgcttg tcggcgatgc ctgcctgcca accagccagc    8460
   catccggccg gccgtccttg acgccttcgc ttccggcgcg gccatcgatt caattcaccc    8520
   atccgatacg ttccgccccc tcacgtccgt ctgcgcacga cccctgcacg accacgccaa    8580
   ggccaacgcg ccgctcagct cagcttgtcg acgagtcgca cgtcacatat ctcagatgca    8640
   tttggactgt gagtgttatt atgccactag cacgcaacga tcttcggggt cctcgctcat    8700
   tgcatccgtt cgggccctgc aggcgtggac gcgagtcgcc gccgagacgc tgcagcaggc    8760
   cgctccgacg cgagggctcg agctcgccgc gcccgcgcga tgtctgcctg gcgccgactg    8820
   atctctggag cgcaaggaag acacggcgac gcgaggagga ccgaagagag acgctggggt    8880
   atgcaggata tacccggggc gggacattcg ttccgcatac actcccccat tcgagcttgc    8940
   tcgtccttgg cagagccgag cgcgaacggt tccgaacgcg gcaaggattt tggctctggt    9000
   gggtggactc cgatcgaggc gcaggttctc cgcaggttct cgcaggccgg cagtggtcgt    9060
   tagaaatagg gagtgccgga gtcttgacgc gccttagctc actctccgcc cacgcgcgca    9120
   tcgccgccat gccgccgtcc cgtctgtcgc tgcgctggcc gcgaccggct gcgccagagt    9180
   acgacagtgg gacagagctc gaggcgacgc gaatcgctcg ggttgtaagg gtttcaaggg    9240
   tcgggcgtcg tcgcgtgcca aagtgaaaat agtagggggg ggggggggta c              9291
<210> 6
   <211> 6175
   <212> DNA
   <213> Artificial Sequence
<220>
   <223> pCL0121
<400> 6
<210> 7
   <211> 7174
   <212> DNA
   <213> Artificial Sequence
<220>
   <223> pCL0125
<400> 7
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg gggggggggg agaggcagga      840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaagaacat 1080
   catctcgctc gtctccaaga agaaggccgc ttccaagaac gaggacaaga acatctcgga 1140
   gtcgtcccgc gacatcgtca accagcagga ggtgtttaac actgaggact ttgaggaggg 1200
   aaagaaggat tccgcttttg agcttgatca cctcgagttt accaccaact cggcccagct 1260
   cggtgatagc gacgaggaca acgagaacgt catcaacgag atgaacgcga cggacgatgc 1320
   taacgaggct aacagcgagg agaagtccat gaccctcaag caggccctcc ttaagtaccc 1380
   gaaggccgcc ctttggtcca tccttgtttc caccactctt gtcatggagg gttacgacac 1440
   cgcccttctc agcgccctct acgctctccc tgtttttcag cgcaagtttg gcaccctcaa 1500
   cggagagggc tcctacgaga tcacctccca gtggcagatc ggtcttaaca tgtgcgtgct 1560
   ctgcggtgag atgatcggtc tccagatcac cacctacatg gttgagttta tgggtaaccg 1620
   ctacactatg attaccgctc tcggcctcct cacggcgtac atctttatcc tctactactg 1680
   caagtccctt gccatgattg ccgtcggtca gattctcagc gccattccct ggggttgctt 1740
   ccagtccctc gccgtcacct acgcctccga ggtctgcccc ctcgcccttc gttactacat 1800
   gacctcctac agcaacattt gctggctgtt tggacagatt tttgcttccg gcatcatgaa 1860
   gaactcccag gagaacctcg gcaacagcga tctcggttac aagctcccct tcgcgctcca 1920
   gtggatttgg ccggcccccc tcatgatcgg cattttcttt gcccccgaga gcccctggtg 1980
   gctcgtgcgt aaggatcgtg tcgccgaggc ccgcaagtcg ctctcccgta ttctctccgg 2040
   caagggcgcc gagaaggaca ttcaggtcga cctcaccctc aagcagattg agcttaccat 2100
   tgagaaggag cgcctcctcg cgtccaagtc gggctccttt tttaactgct ttaagggagt 2160
   caacggccgc cgtacccgtc ttgcctgcct gacctgggtt gcccagaact cgtcgggcgc 2220
   cgtgctcctc ggctactcca cctacttttt tgagaagaag caggtcatgg ccaccgacaa 2280
   ggctttcact ttctcgctta tccagtactg cctcggcctt gccggtactc tctgcagctg 2340
   ggtcatctcg ggccgcgtgg gccgctggac catcctcacg tacggcctcg cttttcagat 2400
   ggtttgcctc tttattatcg gtggcatggg atttggttcg ggctcctccg cttccaacgg 2460
   cgccggcggc ctccttctcg ctctcagctt tttttacaac gccggaattg gtgccgtcgt 2520
   ctactgcatt gttgccgaga tcccttcggc cgagctccgc acgaagacca tcgtccttgc 2580
   ccgcatctgc tacaacctca tggccgtcat caacgccatc ctcaccccct acatgctcaa 2640
   cgtgtcggac tggaactggg gcgccaagac gggactttac tggggtggct ttaccgctgt 2700
   gacccttgcc tgggttatta tcgacctccc cgagaccacc ggccgtacct tctccgagat 2760
   caacgagctc tttaaccagg gtgtccccgc ccgtaagttt gcctcgacgg tcgtcgaccc 2820
   ttttggaaag ggcaagacgc agcacgactc cctcgccgac gagtccattt cgcagtccag 2880
   ctcgatcaag cagcgtgagc ttaacgccgc cgacaagtgc taacatatga gttatgagat 2940
   ccgaaagtga accttgtcct aacccgacag cgaatggcgg gagggggcgg gctaaaagat 3000
   cgtattacat agtatttttc ccctactctt tgtgtttgtc tttttttttt ttttgaacgc 3060
   attcaagcca cttgtctggg tttacttgtt tgtttgcttg cttgcttgct tgcttgcctg 3120
   cttcttggtc agacggccca aaaaagggaa aaaattcatt catggcacag ataagaaaaa 3180
   gaaaaagttt gtcgaccacc gtcatcagaa agcaagagaa gagaaacact cgcgctcaca 3240
   ttctcgctcg cgtaagaatc ttagccacgc atacgaagta atttgtccat ctggcgaatc 3300
   tttacatgag cgttttcaag ctggagcgtg agatcatacc tttcttgatc gtaatgttcc 3360
   aaccttgcat aggcctcgtt gcgatccgct agcaatgcgt cgtactcccg ttgcaactgc 3420
   gccatcgcct cattgtgacg tgagttcaga ttcttctcga gaccttcgag cgctgctaat 3480
   ttcgcctgac gctccttctt ttgtgcttcc atgacacgcc gcttcaccgt gcgttccact 3540
   tcttcctcag acatgccctt ggctgcctcg acctgctcgg taaaacgggc cccagcacgt 3600
   gctacgagat ttcgattcca ccgccgcctt ctatgaaagg ttgggcttcg gaatcgtttt 3660
   ccgggacgcc ggctggatga tcctccagcg cggggatctc atgctggagt tcttcgccca 3720
   ccccaacttg tttattgcag cttataatgg ttacaaataa agcaatagca tcacaaattt 3780
   cacaaataaa gcattttttt cactgcattc tagttgtggt ttgtccaaac tcatcaatgt 3840
   atcttatcat acatggtcga cctgcaggaa cctgcattaa tgaatcggcc aacgcgcggg 3900
   gagaggcggt ttgcgtattg ggcgctcttc cgcttcctcg ctcactgact cgctgcgctc 3960
   ggtcgttcgg ctgcggcgag cggtatcagc tcactcaaag gcggtaatac ggttatccac 4020
   agaatcaggg gataacgcag gaaagaacat gtgagcaaaa ggccagcaaa aggccaggaa 4080
   ccgtaaaaag gccgcgttgc tggcgttttt ccataggctc cgcccccctg acgagcatca 4140
   caaaaatcga cgctcaagtc agaggtggcg aaacccgaca ggactataaa gataccaggc 4200
   gtttccccct ggaagctccc tcgtgcgctc tcctgttccg accctgccgc ttaccggata 4260
   cctgtccgcc tttctccctt cgggaagcgt ggcgctttct catagctcac gctgtaggta 4320
   tctcagttcg gtgtaggtcg ttcgctccaa gctgggctgt gtgcacgaac cccccgttca 4380
   gcccgaccgc tgcgccttat ccggtaacta tcgtcttgag tccaacccgg taagacacga 4440
   cttatcgcca ctggcagcag ccactggtaa caggattagc agagcgaggt atgtaggcgg 4500
   tgctacagag ttcttgaagt ggtggcctaa ctacggctac actagaagaa cagtatttgg 4560
   tatctgcgct ctgctgaagc cagttacctt cggaaaaaga gttggtagct cttgatccgg 4620
   caaacaaacc accgctggta gcggtggttt ttttgtttgc aagcagcaga ttacgcgcag 4680
   aaaaaaagga tctcaagaag atcctttgat cttttctacg gggtctgacg ctcagtggaa 4740
   cgaaaactca cgttaaggga ttttggtcat gagattatca aaaaggatct tcacctagat 4800
   ccttttaaat taaaaatgaa gttttaaatc aatctaaagt atatatgagt aaacttggtc 4860
   tgacagttac caatgcttaa tcagtgaggc acctatctca gcgatctgtc tatttcgttc 4920
   atccatagtt gcctgactcc ccgtcgtgta gataactacg atacgggagg gcttaccatc 4980
   tggccccagt gctgcaatga taccgcgaga cccacgctca ccggctccag atttatcagc 5040
   aataaaccag ccagccggaa gggccgagcg cagaagtggt cctgcaactt tatccgcctc 5100
   catccagtct attaattgtt gccgggaagc tagagtaagt agttcgccag ttaatagttt 5160
   gcgcaacgtt gttgccattg ctacaggcat cgtggtgtca cgctcgtcgt ttggtatggc 5220
   ttcattcagc tccggttccc aacgatcaag gcgagttaca tgatccccca tgttgtgcaa 5280
   aaaagcggtt agctccttcg gtcctccgat cgttgtcaga agtaagttgg ccgcagtgtt 5340
   atcactcatg gttatggcag cactgcataa ttctcttact gtcatgccat ccgtaagatg 5400
   cttttctgtg actggtgagt actcaaccaa gtcattctga gaatagtgta tgcggcgacc 5460
   gagttgctct tgcccggcgt caatacggga taataccgcg ccacatagca gaactttaaa 5520
   agtgctcatc attggaaaac gttcttcggg gcgaaaactc tcaaggatct taccgctgtt 5580
   gagatccagt tcgatgtaac ccactcgtgc acccaactga tcttcagcat cttttacttt 5640
   caccagcgtt tctgggtgag caaaaacagg aaggcaaaat gccgcaaaaa agggaataag 5700
   ggcgacacgg aaatgttgaa tactcatact cttccttttt caatattatt gaagcattta 5760
   tcagggttat tgtctcatga gcggatacat atttgaatgt atttagaaaa ataaacaaat 5820
   aggggttccg cgcacatttc cccgaaaagt gccacctgac gtctaagaaa ccattattat 5880
   catgacatta acctataaaa ataggcgtat cacgaggccc tttcgtctcg cgcgtttcgg 5940
   tgatgacggt gaaaacctct gacacatgca gctcccggag acggtcacag cttgtctgta 6000
   agcggatgcc gggagcagac aagcccgtca gggcgcgtca gcgggtgttg gcgggtgtcg 6060
   gggctggctt aactatgcgg catcagagca gattgtactg agagtgcacc aagcttgagg 6120
   tctgtcgata atccactttt ccattgattt tccaggtttc gttaactcat gccactgagc 6180
   aaaacttcgg tctttcctaa caaaagctct cctcacaaag catggcgcgg caacggacgt 6240
   gtcctcatac tccactgcca cacaaggtcg ataaactaag ctcctcacaa atagaggaga 6300
   attccactga caactgaaaa caatgtatga gagacgatca ccactggagc ggcgcggcgg 6360
   ttgggcgcgg aggtcggcag caaaaacaag cgactcgccg agcaaacccg aatcagcctt 6420
   cagacggtcg tgcctaacaa cacgccgttc taccccgcct tcttcgcgcc ccttcgcgtc 6480
   caagcatcct tcaagtttat ctctctagtt caacttcaag aagaacaaca ccaccaacac 6540
   catggccaag ttgaccagtg ccgttccggt gctcaccgcg cgcgacgtcg ccggagcggt 6600
   cgagttctgg accgaccggc tcgggttctc ccgggacttc gtggaggacg acttcgccgg 6660
   tgtggtccgg gacgacgtga ccctgttcat cagcgcggtc caggaccagg tggtgccgga 6720
   caacaccctg gcctgggtgt gggtgcgcgg cctggacgag ctgtacgccg agtggtcgga 6780
   ggtcgtgtcc acgaacttcc gggacgcctc cgggccggcc atgaccgaga tcggcgagca 6840
   gccgtggggg cgggagttcg ccctgcgcga cccggccggc aactgcgtgc acttcgtggc 6900
   cgaggagcag gactgacacg tgctacgaga tttcgattcc accgccgcct tctatgaaag 6960
   gttgggcttc ggaatcgttt tccgggacgc cggctggatg atcctccagc gcggggatct 7020
   catgctggag ttcttcgccc accccaactt gtttattgca gcttataatg gttacaaata 7080
   aagcaatagc atcacaaatt tcacaaataa agcatttttt tcactgcatt ctagttgtgg 7140
   tttgtccaaa ctcatcaatg tatcttatcg gtac 7174
<210> 8
   <211> 6871
   <212> DNA
   <213> Artificial Sequence
<220>
   <223> pCL0126
<400> 8
   ctcttatctg cctcgcgccg ttgaccgccg cttgactctt ggcgcttgcc gctcgcatcc        60
   tgcctcgctc gcgcaggcgg gcgggcgagt gggtgggtcc gcagccttcc gcgctcgccc       120
   gctagctcgc tcgcgccgtg ctgcagccag cagggcagca ccgcacggca ggcaggtccc       180
   ggcgcggatc gatcgatcca tcgatccatc gatccatcga tcgtgcggtc aaaaagaaag       240
   gaagaagaaa ggaaaaagaa aggcgtgcgc acccgagtgc gcgctgagcg cccgctcgcg       300
   gtcccgcgga gcctccgcgt tagtccccgc cccgcgccgc gcagtccccc gggaggcatc       360
   gcgcacctct cgccgccccc tcgcgcctcg ccgattcccc gcctcccctt ttccgcttct       420
   tcgccgcctc cgctcgcggc cgcgtcgccc gcgccccgct ccctatctgc tccccagggg       480
   ggcactccgc accttttgcg cccgctgccg ccgccgcggc cgccccgccg ccctggtttc       540
   ccccgcgagc gcggccgcgt cgccgcgcaa agactcgccg cgtgccgccc cgagcaacgg       600
   gtggcggcgg cgcggcggcg ggcggggcgc ggcggcgcgt aggcggggct aggcgccggc       660
   taggcgaaac gccgcccccg ggcgccgccg ccgcccgctc cagagcagtc gccgcgccag       720
   accgccaacg cagagaccga gaccgaggta cgtcgcgccc gagcacgccg cgacgcgcgg       780
   cagggacgag gagcacgacg ccgcgccgcg ccgcgcgggg gggggggagg agaggcagga        840
   cgcgggagcg agcgtgcatg tttccgcgcg agacgacgcc gcgcgcgctg gagaggagat       900
   aaggcgcttg gatcgcgaga gggccagcca ggctggaggc gaaaatgggt ggagaggata      960
   gtatcttgcg tgcttggacg aggagactga cgaggaggac ggatacgtcg atgatgatgt     1020
   gcacagagaa gaagcagttc gaaagcgact actagcaagc aagggatcca tggagaacgg     1080
   cactaagcgt gagggccttg gaaagctcac tgtcagctcc tccctccagg tcgagcagcc     1140
   cctcgccccc agcaagctct ggaagattat tgtcgtcgct tcgatcgccg ccggtgtcca     1200
   gtttggctgg gcccttcagc tctcgctcct tactccctac gttcagcttc tcggtattcc     1260
   tcacaagttc gcgtccttta tttggctctg cggaccgatt tcgggtatga tcgtgcagcc     1320
   ggtcgtcggt tactactcgg ataactgctc gtcgcgtttt ggccgtcgcc gtccttttat     1380
   cgccgctggt gccgctctcg ttatgattgc cgttttcctt atcggctttg ccgctgacct     1440
   cggccacgcc tcgggtgaca ccctcggtaa gggtttcaag cctcgcgcca ttgccgtctt     1500
   cgtcgtcggc ttctggattc tcgacgtcgc taacaacatg cttcagggac cctgccgcgc     1560
   cctcctcgct gacctctccg gcggaaagtc cggccgcatg cgcaccgcta acgccttctt     1620
   cagcttcttc atggccgtcg gcaacatcct cggctacgcc gccggctcct acagccacct     1680
   ctttaaggtc tttccctttt ccaagacgaa ggcctgcgat atgtactgcg ctaacctcaa     1740
   gtcctgcttt tttatcgcca tcttccttct tctctcgctc acgaccattg ccctgaccct     1800
   cgtccgcgag aacgagctcc ctgagaagga cgagcaggag atcgacgaga agctcgctgg     1860
   cgccggcaag tccaaggtcc cgttcttcgg cgagatcttt ggcgccctca aggagcttcc     1920
   ccgcccgatg tggattctcc tccttgttac ttgcctcaac tggattgcct ggtttccctt     1980
   tttcctctac gacactgact ggatggctaa ggaggttttt ggtggccagg tcggtgatgc     2040
   ccgcctctac gatctcggcg tccgcgctgg tgcgatgggc cttctccttc agagcgtcgt     2100
   cctcggcttt atgtccctcg gagttgagtt tctcggtaag aagatcggcg gagccaagcg     2160
   cctttggggc atcctcaact ttgtgctcgc catctgcctg gctatgacca tccttgtgac     2220
   taagatggct gagaagtccc gccagcatga tcctgccggc acgcttatgg gtccgacccc     2280
   cggcgtcaag atcggcgctc ttcttctctt tgctgccctc ggtattcccc tcgccgccac     2340
   tttttcgatt cccttcgctc tcgcctcgat tttctcctcc aaccgtggtt cgggtcaggg     2400
   tctcagcctc ggtgtgctca accttgccat tgtggtcccc cagatgcttg tctcgcttgt     2460
   cggcggaccc tgggatgatc ttttcggcgg aggaaacctt ccgggcttcg ttgttggcgc     2520
   tgtcgccgcc gcggcctcgg ctgtcctcgc cctcaccatg ctcccttcgc ctcccgccga     2580
   tgccaagcct gccgttgcca tgggccttag cattaagtaa catatgagtt atgagatccg     2640
   aaagtgaacc ttgtcctaac ccgacagcga atggcgggag ggggcgggct aaaagatcgt      2700
   attacatagt atttttcccc tactctttgt gtttgtcttt tttttttttt tgaacgcatt     2760
   caagccactt gtctgggttt acttgtttgt ttgcttgctt gcttgcttgc ttgcctgctt 2820
   cttggtcaga cggcccaaaa aagggaaaaa attcattcat ggcacagata agaaaaagaa 2880
   aaagtttgtc gaccaccgtc atcagaaagc aagagaagag aaacactcgc gctcacattc 2940
   tcgctcgcgt aagaatctta gccacgcata cgaagtaatt tgtccatctg gcgaatcttt 3000
   acatgagcgt tttcaagctg gagcgtgaga tcataccttt cttgatcgta atgttccaac 3060
   cttgcatagg cctcgttgcg atccgctagc aatgcgtcgt actcccgttg caactgcgcc 3120
   atcgcctcat tgtgacgtga gttcagattc ttctcgagac cttcgagcgc tgctaatttc 3180
   gcctgacgct ccttcttttg tgcttccatg acacgccgct tcaccgtgcg ttccacttct 3240
   tcctcagaca tgcccttggc tgcctcgacc tgctcggtaa aacgggcccc agcacgtgct 3300
   acgagatttc gattccaccg ccgccttcta tgaaaggttg ggcttcggaa tcgttttccg 3360
   ggacgccggc tggatgatcc tccagcgcgg ggatctcatg ctggagttct tcgcccaccc 3420
   caacttgttt attgcagctt ataatggtta caaataaagc aatagcatca caaatttcac 3480
   aaataaagca tttttttcac tgcattctag ttgtggtttg tccaaactca tcaatgtatc 3540
   ttatcataca tggtcgacct gcaggaacct gcattaatga atcggccaac gcgcggggag 3600
   aggcggtttg cgtattgggc gctcttccgc ttcctcgctc actgactcgc tgcgctcggt 3660
   cgttcggctg cggcgagcgg tatcagctca ctcaaaggcg gtaatacggt tatccacaga 3720
   atcaggggat aacgcaggaa agaacatgtg agcaaaaggc cagcaaaagg ccaggaaccg 3780
   taaaaaggcc gcgttgctgg cgtttttcca taggctccgc ccccctgacg agcatcacaa 3840
   aaatcgacgc tcaagtcaga ggtggcgaaa cccgacagga ctataaagat accaggcgtt 3900
   tccccctgga agctccctcg tgcgctctcc tgttccgacc ctgccgctta ccggatacct 3960
   gtccgccttt ctcccttcgg gaagcgtggc gctttctcat agctcacgct gtaggtatct 4020
   cagttcggtg taggtcgttc gctccaagct gggctgtgtg cacgaacccc ccgttcagcc 4080
   cgaccgctgc gccttatccg gtaactatcg tcttgagtcc aacccggtaa gacacgactt 4140
   atcgccactg gcagcagcca ctggtaacag gattagcaga gcgaggtatg taggcggtgc 4200
   tacagagttc ttgaagtggt ggcctaacta cggctacact agaagaacag tatttggtat 4260
   ctgcgctctg ctgaagccag ttaccttcgg aaaaagagtt ggtagctctt gatccggcaa 4320
   acaaaccacc gctggtagcg gtggtttttt tgtttgcaag cagcagatta cgcgcagaaa 4380
   aaaaggatct caagaagatc ctttgatctt ttctacgggg tctgacgctc agtggaacga 4440
   aaactcacgt taagggattt tggtcatgag attatcaaaa aggatcttca cctagatcct 4500
   tttaaattaa aaatgaagtt ttaaatcaat ctaaagtata tatgagtaaa cttggtctga 4560
   cagttaccaa tgcttaatca gtgaggcacc tatctcagcg atctgtctat ttcgttcatc 4620
   catagttgcc tgactccccg tcgtgtagat aactacgata cgggagggct taccatctgg 4680
   ccccagtgct gcaatgatac cgcgagaccc acgctcaccg gctccagatt tatcagcaat 4740
   aaaccagcca gccggaaggg ccgagcgcag aagtggtcct gcaactttat ccgcctccat 4800
   ccagtctatt aattgttgcc gggaagctag agtaagtagt tcgccagtta atagtttgcg 4860
   caacgttgtt gccattgcta caggcatcgt ggtgtcacgc tcgtcgtttg gtatggcttc 4920
   attcagctcc ggttcccaac gatcaaggcg agttacatga tcccccatgt tgtgcaaaaa 4980
   agcggttagc tccttcggtc ctccgatcgt tgtcagaagt aagttggccg cagtgttatc 5040
   actcatggtt atggcagcac tgcataattc tcttactgtc atgccatccg taagatgctt 5100
   ttctgtgact ggtgagtact caaccaagtc attctgagaa tagtgtatgc ggcgaccgag 5160
   ttgctcttgc ccggcgtcaa tacgggataa taccgcgcca catagcagaa ctttaaaagt 5220
   gctcatcatt ggaaaacgtt cttcggggcg aaaactctca aggatcttac cgctgttgag 5280
   atccagttcg atgtaaccca ctcgtgcacc caactgatct tcagcatctt ttactttcac 5340
   cagcgtttct gggtgagcaa aaacaggaag gcaaaatgcc gcaaaaaagg gaataagggc 5400
   gacacggaaa tgttgaatac tcatactctt cctttttcaa tattattgaa gcatttatca 5460
   gggttattgt ctcatgagcg gatacatatt tgaatgtatt tagaaaaata aacaaatagg 5520
   ggttccgcgc acatttcccc gaaaagtgcc acctgacgtc taagaaacca ttattatcat 5580
   gacattaacc tataaaaata ggcgtatcac gaggcccttt cgtctcgcgc gtttcggtga 5640
   tgacggtgaa aacctctgac acatgcagct cccggagacg gtcacagctt gtctgtaagc 5700
   ggatgccggg agcagacaag cccgtcaggg cgcgtcagcg ggtgttggcg ggtgtcgggg 5760
   ctggcttaac tatgcggcat cagagcagat tgtactgaga gtgcaccaag cttgaggtct 5820
   gtcgataatc cacttttcca ttgattttcc aggtttcgtt aactcatgcc actgagcaaa 5880
   acttcggtct ttcctaacaa aagctctcct cacaaagcat ggcgcggcaa cggacgtgtc 5940
   ctcatactcc actgccacac aaggtcgata aactaagctc ctcacaaata gaggagaatt 6000
   ccactgacaa ctgaaaacaa tgtatgagag acgatcacca ctggagcggc gcggcggttg 6060
   ggcgcggagg tcggcagcaa aaacaagcga ctcgccgagc aaacccgaat cagccttcag 6120
   acggtcgtgc ctaacaacac gccgttctac cccgccttct tcgcgcccct tcgcgtccaa 6180
   gcatccttca agtttatctc tctagttcaa cttcaagaag aacaacacca ccaacaccat 6240
   ggccaagttg accagtgccg ttccggtgct caccgcgcgc gacgtcgccg gagcggtcga 6300
   gttctggacc gaccggctcg ggttctcccg ggacttcgtg gaggacgact tcgccggtgt 6360
   ggtccgggac gacgtgaccc tgttcatcag cgcggtccag gaccaggtgg tgccggacaa 6420
   caccctggcc tgggtgtggg tgcgcggcct ggacgagctg tacgccgagt ggtcggaggt 6480
   cgtgtccacg aacttccggg acgcctccgg gccggccatg accgagatcg gcgagcagcc 6540
   gtgggggcgg gagttcgccc tgcgcgaccc ggccggcaac tgcgtgcact tcgtggccga 6600
   ggagcaggac tgacacgtgc tacgagattt cgattccacc gccgccttct atgaaaggtt 6660
   gggcttcgga atcgttttcc gggacgccgg ctggatgatc ctccagcgcg gggatctcat 6720
   gctggagttc ttcgcccacc ccaacttgtt tattgcagct tataatggtt acaaataaag 6780
   caatagcatc acaaatttca caaataaagc atttttttca ctgcattcta gttgtggttt 6840
   gtccaaactc atcaatgtat cttatcggta c 6871
<210> 9
   <211> 6611
   <212> DNA
   <213> Artificial Sequence
<220>
   <223> pCL0122
<400> 9
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc 120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg ggggggaggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaagttcgc 1080
   gacctcggtc gcaattttgc ttgtggccaa catagccacc gccctcgcgc agagcgatgg 1140
   ctgcaccccc accgaccaga cgatggtgag caagggcgag gagctgttca ccggggtggt 1200
   gcccatcctg gtcgagctgg acggcgacgt aaacggccac aagttcagcg tgtccggcga 1260
   gggcgagggc gatgccacct acggcaagct gaccctgaag ttcatctgca ccaccggcaa 1320
   gctgcccgtg ccctggccca ccctcgtgac caccctgacc tacggcgtgc agtgcttcag 1380
   ccgctacccc gaccacatga agcagcacga cttcttcaag tccgccatgc ccgaaggcta 1440
   cgtccaggag cgcaccatct tcttcaagga cgacggcaac tacaagaccc gcgccgaggt 1500
   gaagttcgag ggcgacaccc tggtgaaccg catcgagctg aagggcatcg acttcaagga 1560
   ggacggcaac atcctgggac acaagctgga gtacaactac aacagccaca acgtctatat 1620
   catggccgac aagcagaaga acggcatcaa ggtgaacttc aagatccgcc acaacatcga 1680
   ggacggcagc gtgcagctcg ccgaccacta ccagcagaac acccccatcg gcgacggccc 1740
   cgtgctgctg cccgacaacc actacctgag cacccagtcc gccctgagca aagaccccaa 1800
   cgagaagcgc gatcacatgg tcctgctgga gttcgtgacc gccgccggga tcactctcgg 1860
   catggacgag ctgtacaagc accaccatca ccaccactaa catatgagtt atgagatccg 1920
   aaagtgaacc ttgtcctaac ccgacagcga atggcgggag ggggcgggct aaaagatcgt 1980
   attacatagt atttttcccc tactctttgt gtttgtcttt tttttttttt tgaacgcatt 2040
   caagccactt gtctgggttt acttgtttgt ttgcttgctt gcttgcttgc ttgcctgctt 2100
   cttggtcaga cggcccaaaa aagggaaaaa attcattcat ggcacagata agaaaaagaa 2160
   aaagtttgtc gaccaccgtc atcagaaagc aagagaagag aaacactcgc gctcacattc 2220
   tcgctcgcgt aagaatctta gccacgcata cgaagtaatt tgtccatctg gcgaatcttt 2280
   acatgagcgt tttcaagctg gagcgtgaga tcataccttt cttgatcgta atgttccaac 2340
   cttgcatagg cctcgttgcg atccgctagc aatgcgtcgt actcccgttg caactgcgcc 2400
   atcgcctcat tgtgacgtga gttcagattc ttctcgagac cttcgagcgc tgctaatttc 2460
   gcctgacgct ccttcttttg tgcttccatg acacgccgct tcaccgtgcg ttccacttct 2520
   tcctcagaca tgcccttggc tgcctcgacc tgctcggtaa aacgggcccc agcacgtgct 2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag cttccaattt 5100
   taggcccccc actgaccgag gtctgtcgat aatccacttt tccattgatt ttccaggttt 5160
   cgttaactca tgccactgag caaaacttcg gtctttccta acaaaagctc tcctcacaaa 5220
   gcatggcgcg gcaacggacg tgtcctcata ctccactgcc acacaaggtc gataaactaa 5280
   gctcctcaca aatagaggag aattccactg acaactgaaa acaatgtatg agagacgatc 5340
   accactggag cggcgcggcg gttgggcgcg gaggtcggca gcaaaaacaa gcgactcgcc 5400
   gagcaaaccc gaatcagcct tcagacggtc gtgcctaaca acacgccgtt ctaccccgcc 5460
   ttcttcgcgc cccttcgcgt ccaagcatcc ttcaagttta tctctctagt tcaacttcaa 5520
   gaagaacaac accaccaaca ccatgattga acaagatgga ttgcacgcag gttctccggc 5580
   cgcttgggtg gagaggctat tcggctatga ctgggcacaa cagacaatcg gctgctctga 5640
   tgccgccgtg ttccggctgt cagcgcaggg gcgcccggtt ctttttgtca agaccgacct 5700
   gtccggtgcc ctgaatgaac tgcaggacga ggcagcgcgg ctatcgtggc tggccacgac 5760
   gggcgttcct tgcgcagctg tgctcgacgt tgtcactgaa gcgggaaggg actggctgct 5820
   attgggcgaa gtgccggggc aggatctcct gtcatctcac cttgctcctg ccgagaaagt 5880
   atccatcatg gctgatgcaa tgcggcggct gcatacgctt gatccggcta cctgcccatt 5940
   cgaccaccaa gcgaaacatc gcatcgagcg agcacgtact cggatggaag ccggtcttgt 6000
   cgatcaggat gatctggacg aagagcatca ggggctcgcg ccagccgaac tgttcgccag 6060
   gctcaaggcg cgcatgcccg acggcgatga tctcgtcgtg acccatggcg atgcctgctt 6120
   gccgaatatc atggtggaaa atggccgctt ttctggattc atcgactgtg gccggctggg 6180
   tgtggcggac cgctatcagg acatagcgtt ggctacccgt gatattgctg aagagcttgg 6240
   cggcgaatgg gctgaccgct tcctcgtgct ttacggtatc gccgctcccg attcgcagcg 6300
   catcgccttc tatcgccttc ttgacgagtt cttctgacac gtgctacgag atttcgattc 6360
   caccgccgcc ttctatgaaa ggttgggctt cggaatcgtt ttccgggacg ccggctggat 6420
   gatcctccag cgcggggatc tcatgctgga gttcttcgcc caccccaact tgtttattgc 6480
   agcttataat ggttacaaat aaagcaatag catcacaaat ttcacaaata aagcattttt 6540
   ttcactgcat tctagttgtg gtttgtccaa actcatcaat gtatcttatc atgtctgaat 6600
   tcccggggta c 6611
<210> 10
   <211> 7295
   <212> DNA
   <213> Artificial Sequence
<220>
   <223> pCL0127
<400> 10
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc 120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg ggggggaggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaccaacga 1080
   gacctcggac cgccctctcg tgcactttac ccccaacaag ggttggatga acgatcccaa 1140
   cggcctctgg tacgacgaga aggatgctaa gtggcacctt tactttcagt acaaccctaa 1200
   cgacaccgtc tggggcaccc cgctcttctg ggccacgccc acctccgacg acctcaccaa 1260
   ctgggaggac cagcccattg ctatcgcccc caagcgcaac gactcgggag ctttttccgg 1320
   ttccatggtt gtggactaca acaacacctc cggttttttt aacgacacca ttgacccccg 1380
   ccagcgctgc gtcgccatct ggacctacaa cacgcccgag agcgaggagc agtacatcag 1440
   ctacagcctt gatggaggct acacctttac cgagtaccag aagaaccctg tcctcgccgc 1500
   caactccacc cagttccgcg accctaaggt tttttggtac gagccttccc agaagtggat 1560
   tatgaccgcc gctaagtcgc aggattacaa gatcgagatc tacagcagcg acgacctcaa 1620
   gtcctggaag cttgagtccg cctttgccaa cgagggtttt ctcggatacc agtacgagtg 1680
   ccccggtctc atcgaggtcc ccaccgagca ggacccgtcc aagtcctact gggtcatgtt 1740
   tatttccatc aaccctggcg cccctgccgg cggcagcttc aaccagtact tcgtcggctc 1800
   ctttaacggc acgcattttg aggccttcga caaccagtcc cgcgtcgtcg acttcggcaa 1860
   ggactactac gccctccaga ccttctttaa caccgacccc acctacggca gcgccctcgg 1920
   tattgcttgg gcctccaact gggagtactc cgctttcgtc cccactaacc cctggcgcag 1980
   ctcgatgtcc ctcgtccgca agttttcgct taacaccgag taccaggcca accccgagac 2040
   cgagcttatt aacctgaagg ccgagcctat tctcaacatc tccaacgctg gcccctggtc 2100
   ccgctttgct actaacacta ccctcaccaa ggccaactcc tacaacgtcg atctctccaa 2160
   ctccaccggt actcttgagt ttgagctcgt ctacgccgtc aacaccaccc agaccatctc 2220
   caagtccgtc ttcgccgacc tctccctctg gttcaagggc cttgaggacc ccgaggagta 2280
   cctgcgcatg ggttttgagg tctccgcctc ctccttcttc ctcgatcgcg gtaactccaa 2340
   ggttaagttt gtcaaggaga acccctactt tactaaccgt atgagcgtca acaaccagcc 2400
   ctttaagtcc gagaacgatc ttagctacta caaggtttac ggcctcctcg accagaacat 2460
   tctcgagctc tactttaacg acggagatgt cgtcagcacc aacacctact ttatgaccac 2520
   tggaaacgcc ctcggcagcg tgaacatgac caccggagtc gacaacctct tttacattga 2580
   caagtttcag gttcgcgagg ttaagtaaca tatgagttat gagatccgaa agtgaacctt 2640
   gtcctaaccc gacagcgaat ggcgggaggg ggcgggctaa aagatcgtat tacatagtat 2700
   ttttccccta ctctttgtgt ttgtcttttt tttttttttg aacgcattca agccacttgt 2760
   ctgggtttac ttgtttgttt gcttgcttgc ttgcttgctt gcctgcttct tggtcagacg 2820
   gcccaaaaaa gggaaaaaat tcattcatgg cacagataag aaaaagaaaa agtttgtcga 2880
   ccaccgtcat cagaaagcaa gagaagagaa acactcgcgc tcacattctc gctcgcgtaa 2940
   gaatcttagc cacgcatacg aagtaatttg tccatctggc gaatctttac atgagcgttt 3000
   tcaagctgga gcgtgagatc atacctttct tgatcgtaat gttccaacct tgcataggcc 3060
   tcgttgcgat ccactagcaa tgcgtcgtac tcccgttgca gctgcgccat cgcctcattg 3120
   tgacgtgagt tcagattctt ctcgagacct tcgagcgctg ctaatttcgc ctgacgctcc 3180
   ttcttttgtg cttccatgac acgccgcttc accgtgcgtt ccacttcttc ctcagacatg 3240
   cccttggctg cctcgacctg ctcggtaaaa cgggccccag cacgtgctac gagatttcga 3300
   ttccaccgcc gccttctatg aaaggttggg cttcggaatc gttttccggg acgccggctg 3360
   gatgatcctc cagcgcgggg atctcatgct ggagttcttc gcccacccca acttgtttat 3420
   tgcagcttat aatggttaca aataaagcaa tagcatcaca aatttcacaa ataaagcatt 3480
   tttttcactg cattctagtt gtggtttgtc caaactcatc aatgtatctt atcatacatg 3540
   gtcgacctgc aggaacctgc attaatgaat cggccaacgc gcggggagag gcggtttgcg 3600
   tattgggcgc tcttccgctt cctcgctcac tgactcgctg cgctcggtcg ttcggctgcg 3660
   gcgagcggta tcagctcact caaaggcggt aatacggtta tccacagaat caggggataa 3720
   cgcaggaaag aacatgtgag caaaaggcca gcaaaaggcc aggaaccgta aaaaggccgc 3780
   gttgctggcg tttttccata ggctccgccc ccctgacgag catcacaaaa atcgacgctc 3840
   aagtcagagg tggcgaaacc cgacaggact ataaagatac caggcgtttc cccctggaag 3900
   ctccctcgtg cgctctcctg ttccgaccct gccgcttacc ggatacctgt ccgcctttct 3960
   cccttcggga agcgtggcgc tttctcatag ctcacgctgt aggtatctca gttcggtgta 4020
   ggtcgttcgc tccaagctgg gctgtgtgca cgaacccccc gttcagcccg accgctgcgc 4080
   cttatccggt aactatcgtc ttgagtccaa cccggtaaga cacgacttat cgccactggc 4140
   agcagccact ggtaacagga ttagcagagc gaggtatgta ggcggtgcta cagagttctt 4200
   gaagtggtgg cctaactacg gctacactag aagaacagta tttggtatct gcgctctgct 4260
   gaagccagtt accttcggaa aaagagttgg tagctcttga tccggcaaac aaaccaccgc 4320
   tggtagcggt ggtttttttg tttgcaagca gcagattacg cgcagaaaaa aaggatctca 4380
   agaagatcct ttgatctttt ctacggggtc tgacgctcag tggaacgaaa actcacgtta 4440
   agggattttg gtcatgagat tatcaaaaag gatcttcacc tagatccttt taaattaaaa 4500
   atgaagtttt aaatcaatct aaagtatata tgagtaaact tggtctgaca gttaccaatg 4560
   cttaatcagt gaggcaccta tctcagcgat ctgtctattt cgttcatcca tagttgcctg 4620
   actccccgtc gtgtagataa ctacgatacg ggagggctta ccatctggcc ccagtgctgc 4680
   aatgataccg cgagacccac gctcaccggc tccagattta tcagcaataa accagccagc 4740
   cggaagggcc gagcgcagaa gtggtcctgc aactttatcc gcctccatcc agtctattaa 4800
   ttgttgccgg gaagctagag taagtagttc gccagttaat agtttgcgca acgttgttgc 4860
   cattgctaca ggcatcgtgg tgtcacgctc gtcgtttggt atggcttcat tcagctccgg 4920
   ttcccaacga tcaaggcgag ttacatgatc ccccatgttg tgcaaaaaag cggttagctc 4980
   cttcggtcct ccgatcgttg tcagaagtaa gttggccgca gtgttatcac tcatggttat 5040
   ggcagcactg cataattctc ttactgtcat gccatccgta agatgctttt ctgtgactgg 5100
   tgagtactca accaagtcat tctgagaata gtgtatgcgg cgaccgagtt gctcttgccc 5160
   ggcgtcaata cgggataata ccgcgccaca tagcagaact ttaaaagtgc tcatcattgg 5220
   aaaacgttct tcggggcgaa aactctcaag gatcttaccg ctgttgagat ccagttcgat 5280
   gtaacccact cgtgcaccca actgatcttc agcatctttt actttcacca gcgtttctgg 5340
   gtgagcaaaa acaggaaggc aaaatgccgc aaaaaaggga ataagggcga cacggaaatg 5400
   ttgaatactc atactcttcc tttttcaata ttattgaagc atttatcagg gttattgtct 5460
   catgagcgga tacatatttg aatgtattta gaaaaataaa caaatagggg ttccgcgcac 5520
   atttccccga aaagtgccac ctgacgtcta agaaaccatt attatcatga cattaaccta 5580
   taaaaatagg cgtatcacga ggccctttcg tctcgcgcgt ttcggtgatg acggtgaaaa 5640
   cctctgacac atgcagctcc cggagacggt cacagcttgt ctgtaagcgg atgccgggag 5700
   cagacaagcc cgtcagggcg cgtcagcggg tgttggcggg tgtcggggct ggcttaacta 5760
   tgcggcatca gagcagattg tactgagagt gcaccaagct tgaggtctgt cgataatcca 5820
   cttttccatt gattttccag gtttcgttaa ctcatgccac tgagcaaaac ttcggtcttt 5880
   cctaacaaaa gctctcctca caaagcatgg cgcggcaacg gacgtgtcct catactccac 5940
   tgccacacaa ggtcgataaa ctaagctcct cacaaataga ggagaattcc actgacaact 6000
   gaaaacaatg tatgagagac gatcaccact ggagcggcgc ggcggttggg cgcggaggtc 6060
   ggcagcaaaa acaagcgact cgccgagcaa acccgaatca gccttcagac ggtcgtgcct 6120
   aacaacacgc cgttctaccc cgccttcttc gcgccccttc gcgtccaagc atccttcaag 6180
   tttatctctc tagttcaact tcaagaagaa caacaccacc aacaccatga ttgaacaaga 6240
   tggattgcac gcaggttctc cggccgcttg ggtggagagg ctattcggct atgactgggc 6300
   acaacagaca atcggctgct ctgatgccgc cgtgttccgg ctgtcagcgc aggggcgccc 6360
   ggttcttttt gtcaagaccg acctgtccgg tgccctgaat gaactgcagg acgaggcagc 6420
   gcggctatcg tggctggcca cgacgggcgt tccttgcgca gctgtgctcg acgttgtcac 6480
   tgaagcggga agggactggc tgctattggg cgaagtgccg gggcaggatc tcctgtcatc 6540
   tcaccttgct cctgccgaga aagtatccat catggctgat gcaatgcggc ggctgcatac 6600
   gcttgatccg gctacctgcc cattcgacca ccaagcgaaa catcgcatcg agcgagcacg 6660
   tactcggatg gaagccggtc ttgtcgatca ggatgatctg gacgaagagc atcaggggct 6720
   cgcgccagcc gaactgttcg ccaggctcaa ggcgcgcatg cccgacggcg atgatctcgt 6780
   cgtgacccat ggcgatgcct gcttgccgaa tatcatggtg gaaaatggcc gcttttctgg 6840
   attcatcgac tgtggccggc tgggtgtggc ggaccgctat caggacatag cgttggctac 6900
   ccgtgatatt gctgaagagc ttggcggcga atgggctgac cgcttcctcg tgctttacgg 6960
   tatcgccgct cccgattcgc agcgcatcgc cttctatcgc cttcttgacg agttcttctg 7020
   acacgtgcta cgagatttcg attccaccgc cgccttctat gaaaggttgg gcttcggaat 7080
   cgttttccgg gacgccggct ggatgatcct ccagcgcggg gatctcatgc tggagttctt 7140
   cgcccacccc aacttgttta ttgcagctta taatggttac aaataaagca atagcatcac 7200
   aaatttcaca aataaagcat ttttttcact gcattctagt tgtggtttgt ccaaactcat 7260
   caatgtatct tatcatgtct gaattcccgg ggtac 7295